(19) Europäisches Patentamt
European Patent Office
Office européen des brevets

(11) **EP 2 241 296 B1**

(12) **EUROPÄISCHE PATENTSCHRIFT**

(45) Veröffentlichungstag und Bekanntmachung des Hinweises auf die Patenterteilung:
**14.12.2011 Patentblatt 2011/50**

(51) Int Cl.:
***A61F 13/15*** (2006.01)

(21) Anmeldenummer: **09005461.0**

(22) Anmeldetag: **17.04.2009**

(54) **Inkontinenzartikel in Höschenform**

Incontinence article in the form of underwear

Article d'incontinence en forme de sous-vêtement

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(43) Veröffentlichungstag der Anmeldung:
**20.10.2010 Patentblatt 2010/42**

(73) Patentinhaber: **Paul Hartmann AG**
**89522 Heidenheim (DE)**

(72) Erfinder: **Malowaniec, Krzysztof Daniel**
**89522 Heidenheim (DE)**

(56) Entgegenhaltungen:
**EP-A1- 1 473 008      WO-A1-02/091974**
**US-B1- 6 217 563**

## Beschreibung

Die Erfindung betrifft einen Inkontinenzartikel in Höschenform für die Aufnahme von Körperausscheidungen mit einem vorderen Bauchabschnitt und einem hinteren Rückenabschnitt, die zur Bildung eines in Quer- oder Hirftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in einer Längsrichtung zwischen Bauchabschnitt und Rückenabschnitt erstreckt und an den Bauchabschnitt und an den Rückenabschnitt in Verbindungsbereichen unlösbar angefügt ist, wobei sowohl der Schrittabschnitt als auch der Bauchabschnitt und der Rückenabschnitt die Beinöffnungen des Inkontinenzartikels begrenzen. Ein derart aus drei Komponenten hergestellter Inkontinenzartikel ist beispielsweise aus WO 2004/052260 A1, WO 03/039423 A1, WO 2005/067842 A1, WO 2005/016200 A1 und EP 1 392 212 B1 bekannt. Bei diesem spezifischen Produktkonzept kann nach dem Anfügen des in Längsrichtung erstreckten Schrittabschdnitts an den im Wesentlichen in Quer- oder Hüftumfangsrichtung erstreckten Bauchabschnitt und den entsprechend erstreckten Rückenabschnitt im eben ausgebreiteten Zustand dieser drei Komponenten eine H-förmige Grundstruktur des Inkontinenzartikels realisiert werden. Der Inkontinenzartikel ist dann modular aus den Komponenten Schrittabschnitt, Bauchabschnitt und Rückenabschnitt gebildet. Diese Komponenten werden vorteilhafterweise zunächst über den Schrittabschnitt miteinander verbunden, und vorzugsweise danach wird in beidseitigen Seitennahtbereichen der Bauchabschnitt mit dem Rückenabschnitt verbunden. Hierbei handelt es sich um eine herstellerseitige Verbindung, durch welche die Höschenform erhalten wird. Diese Verbindung ist typischerweise unlösbar. Die Höschenform kann aber auch insbesondere entlang einer Sollbruchlinie, die insbesondere im Seitennahtbereich verlaufen kann, auftrennbar sein, beispielsweise zum Abnehmen eines gebrauchten Inkontinenzartikels bei einer pflegebedürftigen Person.

Inkontinenzartikel in Höschenform unterscheiden sich prinzipiell von traditionellen öffenbaren und schließbaren Inkontinenzartikeln in üblicher Windelform dadurch, dass in der Regel der Hüftumfang vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra-Fäden bezeichnet, in vorgedehntem Zustand (Stretch-Bond-Verfahren) mit Chassismaterialien des Inkontinenzartikels verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen. Der Inkontinenzartikel bzw. die elastifizierten Chassismaterialien des Inkontinenzartikels lassen sich dann wieder elastisch dehnen, wenn der Inkontinenzartikel wie ein Höschen an den Benutzer angelegt wird. Inkontinenzartikel in Höschenform mit derart elastifizierten Chassismaterialien sind mehrfach bekannt geworden und beispielsweise auch in der vorerwähnten WO 2004/052260 A1 behandelt.

Es wurde mit der vorliegenden Erfindung erkannt, dass es beim Anlegevorgang des Inkontinenzartikels in Höschenform, insbesondere beim Eingreifen in die seitlichen Bereiche der Chassismaterialien zu einem Verknittern, teilweise zu bereichsweisen Überfaltungen der mit Elastifizierungsmitteln versehenen Chassismaterialien und damit einhergehend zu einem Verdrillen der Chassismaterialien kommt. Dies stellt eine Beeinträchtigung der elastifizierenden Wirkung und damit der Passform dar, auch der visuelle Eindruck wird unvorteilhaft beeinträchtigt.

Hiervon ausgehend liegt der vorliegenden Erfindung die Aufgabe zugrunde, den vorstehend geschilderten Problemen zu begegnen, ohne dass dies mit einer Beeinträchtigung des Tragekomforts oder weiterer die Funktionalität des Inkontinenzartikels beeinträchtigenden Konsequenzen verbunden ist.

Diese Aufgabe wird nach der Erfindung gelöst durch einen Inkontinenzartikel mit den Merkmalen des Anspruchs 1.

Vorteilhafte Weiterbildungen des Inkontinenzartikels ergeben sich aus den jeweiligen Unteransprüchen.

Die Anordnung der Fügemittel Innerhalb eines ersten und/oder zweiten Fügebereichs kann dabei vollflächig sein.

Die Anordnung der Fügemittel innerhalb eines ersten und/oder zweiten Fügebereichs kann auch nicht vollflächig vorgesehen sein. Fügemittel, welche innerhalb eines Fügebereiches nicht vollflächig angeordnet sind, können vorzugsweise in Form einer punktuellen, einer streifenförmigen oder linienförmigen Anordnung oder einer sonstigen musterartigen Anordnung innerhalb des Fügebereichs vorgesehen sein. Der Fügebereich umfasst im Falle von nicht vollflächig angeordneten Fügemitteln auch die zwischen den Fügemitteln vorhandenen ungebundenen Bereiche. Die Zusammenhörigkeit von nicht vollflächig angeordneten Fügemitteln zu einem Fügebereich wird vorzugsweise durch eine repetitive Anordnung von vorzugsweise gleichen Fügemitteln durch einen Abstand der Fügemittel voneinander von höchstens 10 mm bestimmt. Bei nicht vollflächig angeordneten Fügemitteln wird die flächenhafte Erstreckung des Fügebereiches in Längs- und Querrichtung unter zu Hilfenahme einer imaginären Verbindungslinie durch die jeweils äußeren, also distal gelegenen, und benachbarten Fügemitteln bzw. deren äußersten Randkanten eingegrenzt.

Der durch den zweiten Fügebereich gebildete Verstärkungsbereich hat die für den zweiten Fügebereich in Längsrichtung und Querrichtung ermittelten Abmessungen.

"Unterhalb des Absorptionskörpers" wird dabei als Positionierung einer flächenhaften Erstreckung verstanden, welche in Aufsicht auf einen plan ausgelegten Inkontinenzartikel durch den Absorptionskörpers in Quer- und Längsrichtung überfangen wird. Zudem beschreibt "Fügebereich unterhalb des Absorptionskörpers" die Anordnung des Fügebereiches in Z-Richtung, nämlich zwischen Absorptionkörper und der Innenseite des Bauchabschnitts bzw. Rückenabschnitts.

Unter "Innenseite" des Schrittabschnitts, Bauchabschnitts oder Rückenabschnitts, wird hierbei jeweils die dem Körper des Anwenders zugewandte Oberseite der zu betrachtenden Komponente verstanden. Entsprechend wird unter "Außenseite" jeweils die dem Körper des Anwenders abgewandte und damit der Kleidung zugewandte Oberseite der zu betrachtenden Komponente verstanden.

Die Angaben zu Längen und/oder Breiten des Inkontinenzartikels als solchen oder von definierten Bereichen, wie bspw. der Fügebereiche und dessen Teilbereiche, sind stets auf Abmessungen am Inkontinenzartikel in seinem flach ausgelegten und eben ausgebreiteten Zustand bezogen.

Unter "Überhang" wird hierbei die Erstreckung des Deckblatt-Materials oder des Deckblatt-Materials und des Backsheet-Materials in Querrichtung seitlich außerhalb der Längsränder des Absorptionskörpers verstanden, wobei jeweils die maximale Erstreckung, also die von den Längsrändem des Absorptionskörpers am weitesten distal gelegene äußere Erstreckung des Deckblatt-Materials und/oder des Backsheet-Materials herangezogen wird. Das Backsheet-Material und/oder das Deckblatt-Material kann vorteilhafterweise aus mehreren Komponenten bestehen, so beispielsweise kann das Deckblatt-Material vorteilhafterweise ein Verbund aus einem Topsheet-Material und in Längsrichtung beidseits angrenzenden Barrieremitteln sein. Es wird also so verstanden, dass auch bei Verbunden, also zusammengesetzten Deckblatt-Materialien und/oder Backsheet-Materialien, bei denen die einzelnen Lagen sich nicht kongruent überdecken, bei der Betrachtung des Überhangs die jeweils maximale, also am weitesten distal gelegene äußere Erstreckung des Verbundes bzw. der darin vorkommenden einzelnen Material-Lagen herangezogen wird. Dem Überhang wird jeweils die Breite H zugeordnet.

Unter "Seitenbereich des Bauchabschnitts und/oder Rückenabschnitts" wird hierbei die Erstreckung des Chassismaterials des Bauchabschnitts und/oder Rükkenabschnitts seitlich außerhalb des jeweiligen Längsrandes des Schrittabschnitts in Querrichtung bis zum jeweiligen Längsrandabschnitt des Bauchabschnitts bzw. Rückenabschnitts verstanden. Dabei wird als Längsrand des Schrittabschnitts die maximale Erstreckung, also die am weitesten distal gelegene äußere Erstreckung des Deckblatt-Materials und/oder des Backsheet-Materials, so wie voranstehend erläutert, herangezogen. Dem Seitenbereich des Bauchabschnitts und/oder des Rückenabschnitts wird jeweils die maximal mögliche Breite N zugeordnet. Auch im Falle, dass der schrittseitige und den Beinöffnungen zugewandte Abschnitt des Bauchabschnitts bzw. des Rückenabschnitts eine von der Quer- oder Hüftumfangsrichtung abweichende in Richtung auf eine Quermittelachse des Schrittabschnitts zulaufende Randkontur hat, so insbesondere, wenn diese Randkontur bogenförmig gestaltet ist, wird stets die maximale Breite N herangezogen. D.h. Breite N ist stets der Abstand in Querrichtung gemessen vom Längsrand des Schrittabschnitts bis zum Längsrand des Bauchabschnitts bzw. Rückenabschnitts, welche dann die Seitennahtbereiche des Inkontinenzartikels bilden.

Mit der Erfindung wurde erkannt, dass eine derartige Konstruktion eines Inkontinenzartikels in Höschenform wesentliche Vorteile mit sich bringt:

Durch die Ausbildung eines zweiten Fügebereichs in Form von Schweißstellen werden Verstärkungsbereiche gebildet, die eine nur bereichsweise und im Ausmaß variierbare Versteifung des Überhangs des Schrittabschnitts ermöglichen. Das hat den Vorteil, dass die ersten und/oder zweiten Elastifizierungsmittel zumindest in einem Teilbereich zwischen zwei sich in einer Längsrichtung erstreckenden, versteiften Panels angeordnet sind, welche im Wesentlichen quer, zumindest mit einer signifikanten Komponente in Querrichtung zu den ersten und/oder zweiten Elastifizierungsmitteln verlaufen. Während ein Verstärkungsbereich ein erstes versteiftes Panel bildet, stellt die Seitennaht nämlich für gewöhnlich ein zweites versteiftes Panel dar. Dies wirkt sich positiv vergleichmäßigend auf die Verteilung der die Chassismaterialien in diesem Teilbereich raffenden Rückstellkräfte der ersten und/oder zweiten Elastifizierungsmittel aus. Infolgedessen kann dort eine sehr gleichmäßige Raffung erfolgen und der unerwünschten Überfaltungen der Chassismaterialien und damit einhergehend einem unkontrolliertem Verdrillen der Chassismaterialien entgegengewirkt werden. Somit wirkt sich dies positiv auf die Passform des Inkontinenzartikels aus.

Dadurch, dass die Verbindungsbereiche von Schrittabschnitt mit Bauchabschnitt und mit Rückenabschnitt jeweils entsprechend erste und zweite Fügebereiche umfassen, ist außerdem eine den jeweiligen Bereichen des Inkontinenzartikels bedarfsangepasste Ausführung und Gestaltung der Verbindung zwischen Schrittabschnitt und Bauchabschnitt bzw. Rückenabschnitt möglich:

Es wurde erkannt, dass durch den ersten Fügebereich, welcher zumindest abschnittsweise unterhalb des Absorptionskörpers verläuft, der Absorptionskörper in seiner Positionierung innerhalb des Inkontinenzartikels, insbesondere in Bezug auf die flexiblen Chassismaterialien von Bauch- und Rückenabschnitt, stabilisiert wird. Dies ist vorteilhaft, da beim Anlegevorgang eines Inkontinenzartikels in Höschenform, welcher sich vom Anlegevorgang einer traditionellen Windel des offenen Typs erheblich unterscheidet, Kräfte auf den gesamten Inkontinenzartikel einwirken. Während die Windel des offenen Typs entsprechend plan an den Körper des Anwenders ohne größere Einwirkung von Zugkräften angelegt und dann anschließend verschlossen wird, ist beim Anlegen der herstellerseitig verschlossenen Höschenwindel, bspw. beim Hochziehen zwischen den Beinen, der Absorptionskörper und die damit benachbarten Lagen, durchaus nicht unerheblichen Verformungskräften ausgesetzt. Durch die zumindest abschnittsweise Bindung unterhalb des Absorptionskörpers wird eine nachteilige Verformung und möglicherweise verbleibende wesentliche Verschiebung des Schrittabschnitts zu den Chassismaterialien des Bauch- und Rückenabschnitts unterbunden.

Vorteilhaft ist, dass der zweite Fügebereich dabei sowohl jeweils einen Teilbereich des Überhangs überfängt, und dann über den Längsrand des Schrittteils in den daran angrenzenden Teilbereich des Bauchabschnitts und des Rückenabschnitts übergeht. Dadurch, dass der zweite Fügebereich in einem den Längsrand des Schrittabschnitts überbrückenden Bereich vorgesehen ist, wird der unmittelbare Längsrand des Schrittabschnitts sicher an den Chassismaterialien des jeweiligen Bauch- oder Rückenabschnitts gebunden. Dies ist vorteilhaft, da ein unkontrolliertes Abstehen des Überhargs des Schrittabschnitts und damit eine unkontrollierte Faltenbildung, die zu Hartstellen führen kann, unterbunden werden.

Des Weiteren weist die Ausbildung eines zweiten Fügebereichs mit Fügemitteln in Form von Schweißstellen insbesondere Ultraschallschweißstellen, thermische Schweißstellen und/oder Kalanderschweißstellen einen weiteren Vorteil auf:

Der Einsatz von Klebermaterialien wirft auch bei der maschinellen Fertigung der Inkontinenzartikel die Problematik auf, dass eine äußerst präzise, in schnelllaufenden Windelmaschinen nicht ohne Weiteres machbare Positionierung des Klebermaterials benötigt wird, um ein Austreten der Klebennaterialien über den Randbereich hinaus und damit das Verkleben nicht dafür vorgesehener Bereiche und Materialien innerhalb des Inkontinenzartikels bzw. zwischen mehreren Inkontinenzartikeln zu unterbinden. Der Austritt von Klebemitteln führt außerdem zu Verunreinigung der maschinellen Werkzeuge während der Herstellung des Inkontinenzartikels. Durch den Einsatz von Fügemitteln in Form von Schweißstellen werden die ungewollt produktionstechnisch bedingten Austritte von Klebermaterial vermieden. Zudem wird durch die Fügemittel in Form von Schweißstellen letztlich ein Verstärkungsbereich ohne das Einbringen weiterer zusätzlicher Materialkomponenten erhalten, was Kosten spart.

Mit den Merkmalen des Patentanspruchs 1 wird insgesamt ein Inkontinenzartikel in Höschenform mit dem genannten dreikomponentigen Aufbau geschaffen, bei dem sich eine sichere Verbindung der Komponenten realisieren lässt und die beschriebenen Nachteile des Standes der Technik überwunden sind, und zwar ohne dass dies mit einer Beeinträchtigung des Tragekomforts oder der Funktionalität des Inkontinenzartikels oder seiner Komponenten verbunden wäre, sondern stattdessen die Passform des Inkontinenzartikels deutlich verbessert ist.

In Weiterbildung der Erfindung erweist es sich als vorteilhaft, wenn die zweiten Fügebereiche und damit die Verstärkungsbereiche in Längsrichtung betrachtet beginnend vom schrittzugewandten Querrand des Bauchabschnitts und des Rückenabschnitts sich kontinuierlich bis mindestens zum jeweiligen Längsende des Schrittabschnitts erstrecken.

Die im vorderen und/oder im hinteren Verbindungsbereich entlang des Längsrandes des Schrittabschnitts in Längsrichtung vorgesehenen zweiten Fügebereiche sind vorzugsweise jeweils symmetrisch zueinander angeordnet und weisen vorzugsweise auch jeweils die gleiche Ausgestaltung auf, so dass sie beispielsweise also hinsichtlich Längenerstreckung, Breite, Überlappungsgrad, Art der Fügemittel und/oder Anordnung der Fügemittel und/oder kombinationen davon übereinstimmen.

In Abhängigkeit des Anwendungsbereiches des Inkontinenzartikels und auch der Mobilität des Anwenders kann es vorteilhaft sein, die zweiten Fügebereiche im vorderen und im hinteren Verbindungsbereich unterschiedlich zu gestalten. Vorzugsweise unterscheiden sich die zweiten Fügebereiche im vorderen und hinteren Verbindungsbereich zumindest in einem der Parameter Längenerstreckung, Breite, Überlappungsgrad, Art der Fügemittel und/oder Anordnung der Fügemittel und/oder Kombinationen davon.

In Abhängigkeit des Einsatzbereiches des Inkontinenzartikels und auch der Mobilität des Anwenders kann es vorteilhaft sein, die zweiten Fügebereiche im vorderen und hinteren Verbindungsbereich unterschiedlich in der Längenerstreckung zu gestalten. Weiter vorzugsweise weisen die zweiten Fügebereiche im hinteren Verbindungsbereich eine größere Längenerstreckung auf als im vorderen Verbindungsbereich.

In vorteilhafter Weise ist ein jeweiliger zweiter Fügebereich außerhalb der Kontur des Absorptionskörpers, also außerhalb dessen Längsrändern angeordnet.

In vorteilhafter Weise weist der zweite Fügebereich in dem überfangenen Teilbereich des Überhangs in Querrichtung eine Breite P' von größer 1 mm, vorzugsweise größer 2 mm, weiter vorzugsweise größer 5 mm, aber vorzugsweise von kleiner 60 mm, vorzugsweise kleiner 50 mm, weiter vorzugsweise kleiner 40 mm, weiter vorzugsweise kleiner 30 mm, weiter vorzugsweise kleiner 20 mm auf.

In weiterer vorteilhafter Weise beträgt der Anteil P'/H des durch den zweiten Fügebereich überfangenen Teilbereiches des jeweiligen Überhangs mit der Breite P' bezogen auf den jeweiligen Überhang mit einer Breite H im vorderen und/oder hinteren Überlappungsbereich mindestens 0,01, insbesondere mindestens 0,04, weiter insbesondere mindestens 0,07, weiter insbesondere mindestens 0,10, aber vorzugsweise höchstens 0,90, insbesondere höchstens 0,80, weiter insbesondere höchstens 0,70, weiter insbesondere höchstens 0,60, weiter insbesondere höchstens 0,50, weiter insbesondere höchstens 0,40.

Durch diesen Anteil des Fügebereichs im jeweiligen Überhang kann eine ausreichende Verstärkung der Bindung der chassisbildenden Hüllmaterialien an den Schrittabschnitt erreicht werden, ohne dass die Flexibilität des Überhangs in erheblichem Maße beeinträchtigt wird.

In vorteilhafter Weise weist der jeweilige Überhang eine Breite H vorzugsweise von wenigstens 10 mm, weiter insbesondere von wenigstens 20 mm, weiter insbesondere von 20 bis 100 mm, weiter insbesondere von 20 bis 80 mm auf.

Weiter vorteilhaft überfängt der zweite Fügebereich den an den Längsrand des Schrittabschnitt angrenzenden Teilbereich des Bauchabschnits und/oder Rükkenabschnitts in Querrichtung jeweils mit einer Breite P" von größer 1 mm, vorzugsweise größer 2 mm, weiter vorzugsweise größer 5 mm, aber vorzugsweise von kleiner 60 mm, vorzugsweise kleiner 50 mm, weiter vorzugsweise kleiner 40 mm, weiter vorzugsweise kleiner 30 mm, weiter vorzugsweise kleiner 20 mm.

Insbesondere vorteilhaft beträgt der Anteil P"/H des jeweiligen durch den zweiten Fügebereich überfangenen Teilbereiches des Bauchabschnitts und/oder Rükkenabschnitts mit der Breite P" bezogen auf den jeweiligen Überhang mit einer Breite H mindestens 0,01, insbesondere mindestens 0,04, weiter insbesondere mindestens 0,07, weiter insbesondere mindestens 0,10, aber vorzugsweise höchstens 0,90, insbesondere höchstens 0,80, weiter insbesondere höchstens 0,70, weiter insbesondere höchstens 0,60, weiter insbesondere höchstens 0,50, weiter insbesondere höchstens 0,40.

Vorteilhafterweise weist der zweite Fügebereich und damit der Verstärkungsbereich eine Gesamtbreite P von 5 - 60 mm, insbesondere von 10 - 50 mm, weiter insbesondere von 10 - 40 mm, weiter insbesondere von 10 - 30 mm auf.

Besonders vorteilhaft beträgt der Anteil P"/N des jeweiligen durch den zweiten Fügebereich überfangenen Teilbereiches des Bauchabschnitts und/oder Rükkenabschnitts mit der Breite P" bezogen auf den jeweiligen Seitenbereich des Bauchabschnitts und/oder Rükkenabschnitts mit einer Breite N mindestens 0,01, insbesondere mindestens 0,015, weiter insbesondere mindestens 0,020, aber vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10.

Durch diesen Anteil an zweitem Fügebereich und damit Verstärkungsbereich im jeweiligen Seitenbereich des Bauchabschnitts und/oder Rückenabschnitts kann eine ausreichende Verstärkung erreicht werden, ohne dass diese Chassismaterialien und ihre darin festgelegten Elastifizierungsmittel in ihrer Flexibilität und auch ihrer Wirkungsweise großflächig beeinträchtigt werden.

In einer vorteilhaften Weise beträgt der Anteil P'/N des jeweiligen durch den zweiten Fügebereich überfangenen Teilbereiches des jeweiligen Überhangs mit der Breite P' bezogen auf den jeweiligen Seitenbereich des Bauchabschnitts und/oder des Rückenabschnitts mit einer Breite N mindestens 0,01, insbesondere mindestens 0,015, weiter insbesondere mindestens 0,020, aber vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10.

In vorteilhafter Weise erstreckt sich ein jeweiliger Seitenbereich des Bauchabschnitts und/oder des Rückenabschnitts in Querrichtung mit einer Breite N von vorzugsweise wenigstens 100 mm, weiter vorzugsweise von wenigstens 120 mm, und insbesondere 120 mm bis 350 mm, weiter insbesondere 120 bis 320 mm.

In vorteilhafter Weise ist der zweite Fügebereich mit der Gesamtbreite P derart entlang des Längsrandes des Schrittabschnitts angeordnet, so dass das Verhältnis der Breite P' zur Breite P" vorzugsweise zwischen 1: 4 und 4:1, weiter vorzugsweise zwischen 1:3 und 3:1, weiter vorzugsweise zwischen 1:2 und 2:1, besonders bevorzugt 1: 1 beträgt.

In einer weiteren vorteilhaften Weise ist der zweite Fügebereich parallel zur Längsrichtung mit einer gleich bleibenden Gesamtbreite P angeordnet. Hierdurch kann der zweite Fügebereich mit geringem technischen Aufwand und damit auch schneller und kostengünstiger in den Inkontinenzartikel eingebracht werden.

In weiterer vorteilhafter Weise sind die Fügemittel im zweiten Fügebereich ausgehend von der Innenseite des Schrittabschnitts und des entsprechenden Bauchabschnitts und/oder Ruckenabschnitts eingebracht, derart, dass die durch die Fügemittel, nämlich die Schweißstellen erhaltenen gebundenen Flächen ausgehend von der Innenseite in Richtung Außenseite orientiert sind. Die Fügemittel sind insbesondere aber derart eingebracht, dass die Schweißstellen nicht auf die Außenseite des Bauchabschnitt und/oder Rückenabschnitts durchgedrückt und damit nicht oder kaum erkennbar sind.

In einer vorteilhaften Weiterbildung sind die Fügemittel im zweiten Fügebereich vollflächig gestaltet. Derart kann der zweite Fügebereich mit geringem technischen Aufwand und damit auch kostengünstig in den Inkontinenzartikel eingebracht werden.

Bei dieser bevorzugten Ausführungsform hat die durch die Fügemittel gebundene Fläche einen Anteil von 100% der durch den zweiten Fügebereich umfangenen Gesamtfläche.

In einer weiteren vorteilhaften Weiterbildung sind die Fügemittel im zweiten Fügebereich nicht vollflächig ausgestaltet. Insbesondere sind die Fügemittel innerhalb des zweiten Fügebereiches in Form eines Musters, insbesondere in Form einer punktförmigen und/oder streifenförmigen und/oder linienförmigen Anordnung und/oder einer sonstigen musterartigen Anordnung und/oder in Kombinationen davon, vorgesehen. Hierdurch lässt sich das Ausmaß der Versteifung der zweiten Fügebereiche vorteilhaft einstellen. Wie oben beschrieben wirkt sich die Versteifung vorteilhaft auf die Passform aus; eine zu hohe Versteifung kann jedoch zu als unangenehm empfundenen Hartstellen führen.

In weiterer vorteilhafter Weise nehmen die durch die im zweiten Fügebereich nicht vollflächig angeordneten Fügemittel in Summe eine gebundene Fläche (Fügestellen) mit einem Anteil von mindestens 1,5 %, insbesondere mindestens 2,0%, weiter insbesondere mindestens 2,5% und vorzugsweise von höchstens 60%, insbesondere höchstens 50%, weiter insbesondere höchstens 40%, weiter insbesondere höchstens 30%, weiter insbesondere höchstens 20% bezogen auf die vom zweiten Fügebereich überfangene Gesamtfläche ein.

In besonders vorteilhafter Weise sind die Fügemittel im zweiten Fügebereich in einem Punktmuster angeordnet und die Summe der durch die Fügemittel gebundenen Fläche (Fügestellen) nimmt einen Anteil von mindestens 1,5%, insbesondere mindestens 2,0%, weiter insbesondere mindestens 2,3%, weiter insbesondere mindestens 2,5 %, und vorzugsweise höchstens 20,0%, insbesondere höchstens 15%, weiter insbesondere höchstens 10,0%, weiter insbesondere höchstens 8,0%, weiter insbesondere höchstens 7,0%, weiter insbesondere höchstens 6,0% bezogen auf die vom zweiten Fügebereich übertangene Gesamtfläche ein.

In weiterer vorteilhafter Weise weisen die durch die einzelnen Fügemittel in einem Punktmuster im zweiten Fügebereich gebundenen Flächen einen Durchmesser von mindestens 0,2 mm, insbesondere von mindestens 0,3 mm, weiter insbesondere von mindestens 0,4 mm, weiter insbesondere von mindestens 0,5 mm und vorzugsweise von höchstens 2,5 mm, insbesondere von höchstens 2,0 mm, weiter insbesondere von höchstens 1,5 mm, weiter insbesondere von höchstens 1,2 mm, weiter insbesondere von höchstens 1,0 mm auf.

Insbesondere sind die benachbarten einzelnen in einem Punktmuster vorhandenen Fügemittel jeweils in einem Abstand von 1 - 10 mm, insbesondere von 1 - 8 mm, weiter insbesondere von 1 - 6 mm, weiter insbesondere von 1 - 5 mm, weiter insbesondere von 1,5 - 4,5 mm, weiter insbesondere von 2 - 4 mm, besonders vorzugsweise in einem gleichen Abstand, voneinander angeordnet.

Für einen ersten Fügebereich können vorteilhafterweise nicht-adhäsive Fügemittel, insbesondere entnommen aus der Gruppe von Schweißstellen, weiter insbesondere Ultraschallschweißstellen, thermische Schweißstellen und/oder Kalanderschweißstellen eingesetzt werden. Es hat sich indes als besonders vorteilhaft erwiesen, als Fügemittel in einem ersten Fügebereich ein Klebemittel, insbesondere einen Hotmeltkleber einzusetzen. Weiter insbesondere weist der Kleber bzw. Hotmeltkleber hydrophobe Eigenschaften auf. Dies ist vorteilhaft, da zusätzlich zur verbindenden Funktion gleichzeitig eine Flüssigkoftsbarriere gebildet wird.

Im ersten Fügebereich kann der Schrittabschnitt vorteilhafterweise mittels eines nicht vollflächigen Kleberauftrags mit dem Bauchabschnitt und/oder mit dem Rückenabschnitt verbunden sein. Bei einem nicht vollflächigen Kleberauftrag kann es sich beispielsweise um ein streifenförmiges Muster, um eine stegförmige kontinuierliche oder nicht kontinuierliche Gitterstruktur oder um inselförmige Bereiche oder aber um eine streifenförmige oder spiralförmig angeordnete Kleberstruktur handeln.

Es hat sich indes als besonders vorteilhaft erwiesen, im ersten Fügebereich einen vollflächigen Kleberauftrag einzusetzen. Dadurch wird eine optimierte Bindung des Schrittabschnitts mit einem an sich verwindungssteifen Absorptionskörper an die vielmehr flexiblen Chassismaterialien des Bauchabschnitts und/oder Rückenabschnitts geschaffen und eine unerwünschte Verschiebung der Komponenten zueinander unterbunden.

Vorteilhafterweise erstreckt sich der erste Fügebereich unterhalb des Absorptionskörpers mindestens bis zu den Längsrändern des Absorptionskörpers, endet aber vor den Längsrändern des Schrittabschnitts. Weiter vorteilhafterweise erstreckt sind der erste Fügebereich in Querrichtung über die Längsränder des Absorptionskörpers hinaus, derart dass eine Überlappung mit dem jeweiligen zweiten Fügebereich erhalten wird.

Die chassisbildenden Materialien von Bauchabschnitt und/oder Rückenabschnitt umfassen vorzugsweise Vliesstoffmaterialien, wie Spinnvliese (S), Meltblownvliese (M), SM-Vliese, SMS-Vliese, SMMS-Vliese, Kardenvliese oder Through Air bonded Kardenvliese. Besonders bevorzugt umfasst das chassisbildende Material von Bauchabschnitt und/oder Rückenabschnitt Spinnvlies. Die für Bauchabschnitt und/oder Rückenabschnitt eingesetzten Vliesstoffmaterialien haben vorteilhafterweise ein Flächengewicht von 10 - 30 g/m$^2$, weiter vorzugsweise von 15 - 25 g/m$^2$. Besonders bevorzugt umfassen der Bauchabschnitt und der Rückenabschnitt ein Spinnvlies aus Polypropylen, insbesondere mit einem Flächengewicht von 15 - 25 g/m$^2$.

Für die Ausbildung des Schrittabschnitts wird vorzugsweise ein Backsheet-Material bzw. ein Deckblatt-Material mit geringen Flächengewichten, nämlich von 10-40 g/m$^2$ bzw. 5 -20 g/m$^2$ eingesetzt. Damit wird vorteilhaft die für den Anwender des Inkontinenzartikels in diesen empfindlichen Körperregionen gewünschte Weichheit, Anpassbarkeit und Drapierbarkeit realisiert.

Die chassisbildenden Hüllmaterialien des Schrittabschnitts sind weiter vorteilhaft ausgebildet:
Das Backsheet umfasst insbesondere eine Folie, insbesondere eines Flächengewichts von 10 - 40 g/m$^2$. Insbesondere umfasst das Backsheet eine im Gebrauch flüssigkeitsdichte aber gleichwohl atmungsaktive, also wasserdampfdurchlässige, insbesondere mikroporöse Folie. Die Wasserdampfdurchlässigkeit des Backsheets beträgt insbesondere wenigstens 300 g/m²/24h, weiter insbesondere wenigstens 1000 g/m²/24h, weiter insbesondere wenigstens 2000 g/m²/24h, weiter insbesondere wenigstens 3000 g/m²/24h, weiter insbesondere wenigstens 4000 g/m²/24h, weiter insbesondere höchstens 6000 g/m²/24h gemessen nach DIN 53 122-1 (Ausgabe: 2001-08).

Die Folie kann vorteilhaft auch mit einer Vliesbeschichtung versehen sein, was eine textile Anmutung der körperabgewandten Außenseite des Inkontinenzartikels vermitteln kann. Die Vliesbeschichtung besteht vorzugsweise aus einem Vliesstoff, insbesondere einem Spinnvlies aus Polypropylen, insbesondere mit einem Flächengewicht von 7-25 g/m², 10 - 20 g/m², insbesondere von 12 -17 g/m².

Das Deckblatt-Material umfasst vorzugsweise Vliesstoffmaterialien, wie Spinnvliese (S), Meltblownvliese (M), SM-Vliese, SMS-Vliese, SMMS-Vliese, Kardenvliese oder Through Air bonded Kardenvliese

Das Deckblatt-Material kann dabei vorzugsweise nur aus Topsheet-Material gebildet sein. Weiter vorzugsweise kann das Deckblatt-Material ein Verbund aus Topsheet-Material und Barrieremittel sein. In einer weiteren vorteilhaften Ausbildung ist das Deckblatt-Material ein Verbund aus einem flüssigkeitsdurchlässigen Topsheet-Material mit Längsrändern und angrenzenden Längsrandbereichen und beidseits an den Längsrändern bzw. Längsrandbereichen des Topsheet-Materials in Fügestellen angefügten hydrophoben Barrieremitteln. Durch diesen Verbund werden in einem Inkontinenzartikel die bereichsweise unterschiedlichen Anforderungsprofile, nämlich im mittigen Bereich eine Flüssigkeitsaufnahme und an den Randbereichen eine Retardierung des seitlichen Flüssigkeitsaustritts, bereitgestellt.

Entsprechend der Funktionalität werden nachstehend genannte vorteilhafte Materialien eingesetzt:

Das Topsheet-Material umfasst vorzugsweise Vliesstoffmaterialien, wie Spinnvliese, Kardenvliese oder Through Air bonded Kardenvliese. Besonders bevorzugt umfasst das Topsheet-Material Spinnvlies. Die für das Topsheet eingesetzten Vliesstoffmaterialien haben weiter vorteilhaflerweise ein Flächengewicht von 5 - 20 g/m². 8 - 20 g/m², weiter vorzugsweise von 10 - 18 g/m². insbesondere vorzugsweise von 12 - 16 g/m². Besonders bevorzugt umfasst das Topsheet ein hydrophilisiertes Spinnvlies, insbesondere aus Polypropylen, insbesondere mit einem Flächengewicht von 12 - 16 g/m².

Das Barrieremittel-Material umfasst vorzugsweise Vliesstoffmaterialien, wie Spinnvliese, Meltblownvliese, Kardenvliese oder Through Air bonded Kardenvliese. Besonders bevorzugt umfasst das Barrieremittel-Material einlagige oder mehrlagige Vliese. Insbesondere bevorzugt umfasst das Barrieremittel-Material Laminate aus einer oder mehreren Spinnvlies(S) und/ oder Meltblown(M)-Vlieslagen, insbesondere SMS-Laminate oder SMMS-Laminate, insbesondere auf Basis von Polyolefinen, wie beispielsweise Polyethylen oder Polypropylen. Derartige Materialien sind kostengünstig und aufgrund ihrer inhärent hydrophoben Eigenschaft geeignet, flüssigkeitsretardierend zu wirken.

Die für das Barrieremittel eingesetzten Vliesstoffmaterialien haben weiter vorteilhafterweise ein Flächengewicht von 5 - 20 g/m², vorzugsweise von 8-20 g/m², weiter vorzugsweise von 10-18 g/m². Besonders bevorzugt umfasst das Barrieremittel ein Laminat aus Spinnvlies und Meltblown-Vlieslagen, insbesondere aus Polypropylen, insbesondere mit einem Flächengewicht von 10 - 18 g/m².

In einer weiteren Ausführung erstreckt sich das hydrophobe Barrieremittel über die Längsränder des Topsheet-Materials hinaus, und zwar unter Ausbildung eines jeweils beidseits des Absorptionskörpers in Längsrichtung verlaufenden jeweils aufstehenden Barrieremittels, welches typischerweise als Cuffelement oder Bündchenelement bezeichnet wird. Die distalen Enden der Barrieremittel sind vorteilhafterweise mit Elastifizierungsmitteln versehen. Damit werden die Barrieremittel im Gebrauch des Inkontinenzartikels gegen die Hautoberfläche des Benutzers angehoben.

Die Fixierung der Materialbahnen des Deckblatt-Material-Verbundes in den Fügestellen kann vorzugsweise mittels Kleber, insbesondere Hotmeltkleber, thermisches Kalandern (Thermobonding) oder Ultraschallschweißen erfolgen. Die Fixierung kann in Form von kontinuierlichen Fügestellen ausgeführt sein, um eine hohe Verbundkraft zwischen dem Topsheet-Material und dem Barrieremittel zu erzielen. Denkbar ist dabei eine durchgehende Linie. Denkbar und vorteilhaft ist jedoch auch eine Fixierung durch intermittierend angebrachte Fügestellen, also durch eine Abfolge von diskreten Bindepunkten oder Bindelinien oder jedem anderen Bindemuster.

Vorteilhafterweise haben das Backsheet-Material und das Deckblatt-Material dieselbe Erstreckung in Querrichtung. Sie sind kongruent, also deckungsgleich zueinander.

Weiter vorteilhaft ist aber auch, wenn das Backsheet-Material und Deckblatt-Material nicht kongruent zueinander sind. Insbesondere vorteilhafterweise hat das Backsheet-Material eine im Vergleich zum Deckblatt-Material schmälere Erstreckung in Querrichtung. Damit wird das den Anwender im Tragekomfort möglicherweise störende Backsheet-Material, wie beispielsweise eine Folie, durch das hautfreundliche Vliesmaterial des Deckblatt-Materials überdeckt.

In Weiterbildung der Erfindung erweist es sich als vorteilhaft, wenn der Anteil der Fläche des Schrittabschnitts an der gesamten Fläche des Inkontinenzartikels 25 -55 %, insbesondere 30 - 47 %, weiter insbesondere 35 - 47 % und weiter insbesondere 35 - 45 % beträgt.

Der Überlappungsbereich zwischen Schrittabschnitt und Bauchabschnitt ist in Weiterbildung der Erfindung so ausgebildet, dass der Schrittabschnitt 15 - 40 %, insbesondere 15 - 35 % und weiter insbesondere 15 - 25 % der Fläche des Bauchabschnitts überlappt. In vorteilhafter Weise überlappt der Schrittabschnitt den Bauchabschnitt mit einer Fläche von 25.000 - 45.000 mm².

Der Überlappungsbereich von Schrittabschnitt und Rückenabschnitt ist in Weiterbildung der Erfindung so ausgebildet, dass der Schrittabschnitt 20 - 40 %, insbesondere 20 - 35 % und weiter insbesondere 22 - 32 % der Fläche des Rückenabschnitts überlappt. In vorteilhafter Weise überlappt der Schrittabschnitt den Rückenabschnitt mit einer Fläche von 35.000 - 65.0000 mm², insbesondere von 40.000 - 55.000 mm².

Die Überlappung des Schrittabschnitts mit dem Rückenabschnitt ist in vorteilhafter Weise größer als die Überfappung des Schrittabschnitts mit dem Bauchabschnitt.

Es ist bei der erfindungsgemäßen Ausbildung des Inkontinenzartikels möglich und vorteilhaft, wenn auch der Absorptionskörper 5 - 20%, insbesondere 5 -15 % der Fläche des Bauchabschnitts und/oder 10 - 20 %, insbesondere 10 -15 % der Fläche des Rückenabschnitts überlappt.

Die Erstreckung des Bauchabschnitts und des Rückenabschnitts im Seitennahtbereich in Längsrichtung beträgt vorteilhafterweise wenigstens 100 mm, insbesondere wenigstens 150 mm und insbesondere 150 mm bis 220 mm.

Der minimale Abstand des Bauchabschnitts und des Rückenabschnitts in Längsrichtung voneinander beträgt vorteilhafterweise 250 bis 400 mm.

Die maximale Erstreckung des Schrittabschnitts in Querrichtung, also die größte Breite E, beträgt vorteilhafterweise wenigstens 200 mm, insbesondere 200 bis 350 mm, weiter insbesondere 250 bis 320 mm.

Des weiteren erweist es sich als vorteilhaft, wenn der Überhang des Backsheet-Materials und/oder des Deckblatt-Materials in Querrichtung in der Summe, also beidseits der Längsränder des Absorptionskörpers wenigstens 25%, insbesondere 25 - 50 %, weiter insbesondere 30 - 45 % und weiter insbesondere 35 - 45 % bezogen auf die größte Breite E des Schrittabschnitts beträgt. Der verhältnismäßig große überhang von Backsheet-Material und/oder Deckblatt-Material beidseits der Absorptionskörpers bedeutet also einen breiten Schrittabschnitt mit einem verhältnismäßig schmalen Absorptionskörper. Hierdurch ist es möglich, entlang der Beinöffnungen erstreckte Beinelastifizierungsmittel in dem Schrittabschnitt vorzusehen, die einen verhältnismäßig groBen Abstand zum materialreichen und damit biegesteifen Absorptionskörper haben. Dies resultiert wiederum in einer guten Abdichtbarkeit und Anpassbarkeit der beidseitigen Beinöffnungsränder des Schrittabschnitts. Solchenfalls behindert nämlich der materialreiche und gegenüber dünnen Chassismaterialien verwindungssteife Absorptionskörper die Ausbildung eines flüssigkeitsdichten Beinabschlusses nur wenig; es muss daher zur Ausbildung eines flüssigkeitsdichten Beinabschlusses nicht mit extrem hohen Spannungen gearbeitet werden, was sich wiederum positiv auf den Tragekomfort des Inkontinenzartikels auswirkt.

In noch weitergehender Ausbildung der Erfindung erweist es sich als besonders vorteilhaft, wenn die Beinelastifizierungsmittel in Längsrichtung wenigstens 10 mm, insbesondere wenigstens 20 mm vor den zweiten Elastifizierungsmitteln enden. Insbesondere vorteilhaft ist, wenn die Beinelastifizierungsmittel in Längsrichtung vor dem Bauchabschnitt und/oder vor dem Rückenabschnitt enden. Die von ihnen ausgeübte Spannung und Rückstellkraft beeinflusst daher nicht Spannungsverhältnisse der zweiten Elastifizierungsmittel. Insbesondere werden nicht die Spannungsverhältnisse innerhalb des schrittseitigen und den Beinöffnungen zugewandten Bereichs des Bauchabschnitts und des Rückenabschnitts, in dem die zweiten Elastifizierungsmittel sich auffächernden vorgesehen sind, beeinflusst.

Als Beinelastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®-, Creora®- oder Spandex®-Fäden eingesetzt. Die Beinelastifizierungsmittel haben vorzugsweise eine Stärke von 300 - 1500 dtex, insbesondere von 500 - 1200 dtex, weiter insbesondere von 500 - 900 dtex. Die Beinelastifizierungsmittel werden vorzugsweise unter einer Vorspannung von 1,5 - 6,0, insbesondere von 2,5 - 4,5 an den chassisbildenden Hüllmaterialien des Schrittabschnitts fixiert. Die Vorspannung ist definiert als Faktor des Dehnungsgrades gegenüber dem ungedehnten/relaxierten Zustand des Elastifizierungsmittels.

Zur flächenhaften Elastifizierung von Bauchabschnitt und Rückenabschnitt sind die jeweils in einem Abstand voneinander und parallel zueinander in Quer- oder Hüftumfangsrichtung erstreckten ersten Elastifizierungsmittel vorgesehen. Diese haben vorzugsweise gleiche Vorspannung und dienen im Wesentlichen einer flächenhaft durchgehenden gleichmäßigen Elastifizierung des Bauchabschnitts und des Rückenabschnitts in dem Bereich deutlich oberhalb der Beinöffnungen. Es ist indessen möglich, dass die ersten Elastifizierungsmittel in einem oberen Hüftrandbereich eine stärkere Vorspannung aufweisen oder mehrere dieser Elastifizierungsmittel mit geringerern Abstand voneinander vorgesehen sind, um eine etwas stärkere Elastifizierung am Hüftrand zu realisieren.

In noch weitergehender Ausbildung der vorliegenden Erfindung wurde auch erkannt, dass die Spannungsverhältnisse in dem genannten schrittseitigen und den Beinöffnungen zugewandten Bereich des Bauchabschnitts und des Rückenabschnitts im Hinblick auf den Tragekomfort wesentlich sind und so gestaltet werden können, dass der Tragekomfort verbessert wird. Vorteilhafterweise erstrecken sich die zweiten Elastifizierungsmittel ausgehend von den beiden Seitennahtbereichen in Richtung auf eine Längsmittelachse des Inkontinenzartikels und verlaufen dabei bogenförmig sich auffächernd mit zunehmendem Abstand voneinander.

Hierfür ist der schrittseitige und den Beinöffnungen zugewandte Bereich, in welchem die zweiten Elastifizierungsmittel sich in Richtung auf die Längsmittelachse auffächern, vorzugsweise so ausgebildet, dass bei flächenhafter Dehnung dieses Bereichs die dabei auftretende Rückstellkraft in Richtung auf den Schrittabschnitt abnimmt.

Betrachtet man also diesen schnittseitigen und den Beinöffnungen zugewandten Bereich des Bauchabschnitts und des Rückenabschnitts, und zwar in einer Richtung ausgehend von dem jeweiligen Seitennahtbereich in Richtung auf den Schrittabschnitt, also in Richtung auf eine Längsmittelachse des Inkontinenzartikels und gewissermaßen in Richtung der bogenförmigen Auffächerung der zweiten Elastifizierungsmittel, so wird die bei flächenhafter Dehnung auftretende Rückstellkraft in dieser Richtung reduziert. Es handelt sich also hierbei um diejenige Kraft, welche der Bauchabschnitt und der Rückenabschnitt einer flächenhaften Dehnung entgegensetzen. Eine Abnahme dieser Rückstellkraft, die sich dann naturgemäß auf den Benutzer überträgt, ist mit einer erheblichen Verbesserung des Tragekomforts des Inkontinenzartikels verbunden.

Es erweist sich weiter als besonders vorteilhaft, wenn die Abnahme der Rückstellkraft in dem genannten schrittseitigen und den Beinöffnungen zugewandten Bereich des Bauchabschnitts und des Rückenabschnitts so vorgesehen wird, dass in Richtung auf den Schrittabschnitt eine abnehmende Anzahl von Falten pro cm-Querrichtung des Inkontinenzartikels gebildet wird. Solchenfalls können sich der Bauchabschnitt und der Rückenabschnitt entsprechend der Körperform des Benutzers dehnen, ohne dass die hierdurch gebildeten elastischen Kräfte das Chassismaterial mit einer Vielzahl von Falten zu raffen versuchen. Es sei an dieser Stelle nochmals erläutert, dass die Abnahme der Rückstellkraft in Richtung auf den Schrittabschnitt bedeutet, dass diejenige Kraft, die infolge einer flächenhaften Dehnung erzeugt wird, mit zunehmender Annäherung an den Schrittabschnitt geringer wird. Die Rückstellkraft infolge flächenhafter Dehnung ist also in einem Gebiet näher der Seitennaht größer als in einem Gebiet näher dem Schrittabschnitt.

Die genannten Spannungsverhältnisse lassen sich in verschiedenster Weise erreichen, etwa durch Verwendung von in Querrichtung unterschiedlich elastischen Materialien in dem schrittseitigen und den Beinöffnungen zugewandten Bereich, in welchem auch die zweiten Elastifizierungsmittel vorgesehen sind. Es wäre auch denkbar, dass die Vorspannung der zweiten Elastifizierungsmittel mit zunehmender Annäherung an den Schrittabschnitt, also von außen nach innen in Richtung auf eine Längsmitttelachse reduziert wird. Außerdem wäre es denkbar, dass die Abnahme der Rückstellkraft bei flächenhafter Dehnung dadurch erreicht wird, dass der Abstand der zweiten Elastifizierungsmittel voneinander zunimmt, wobei hier darauf zu achten ist, dass dies nicht mit einer starken Zunahme der Vorspannung infolge des fächerförmigen Verlaufs der Elastifizierungsmittel ausgeglichen oder gar in Richtung zunehmender Rückstellkraft übertroffen wird.

Zur Bestimmung der Rückstellkräfte können die zu vermessenden Bereiche des Chassis direkt, gleichsam zerstörungsfrei zwischen zwei Klemmbacken von definierter, gleicher Klemmbackenbreite fest eingespannt und die Rückstellkräfte bei einer definierten, den Gebrauchszustand simulierenden Dehnung der zu messenden Bereiche um insbesondere 30% oder 50% oder 80% der Ausgangslänge (des Klemmbackenabstandes bei Fixieren des zu messendes Bereiches in ungespanntern Zustand) ermittelt werden. Die Klemmbacken solten möglichst viele, zumindest jedoch zwei nebeneinander angeordnete Elastifizierungsmittel des zu messenden Bereiches fixieren und im Wesentlichen senkrecht zum Verlauf der Elastifizierungsmittel orientiert sein, so dass die Dehnung zwischen den Klemmen im Wesentlichen in Richtung des Verlaufs der Elastifizierungsmittel erfolgt.

Es hat sich insbesondere als vorteilhaft erwiesen, wenn ein minimaler Abstand der zweiten Elastifizierungsmittel voneinander (Abstand von unmittelbar nebeneinander liegenden Elastifizierungsmitteln) in den Seitennahtbereichen 3 bis 8 mm, insbesondere 3 bis 7 mm und weiter insbesondere 3 bis 6 mm beträgt.

Des Weiteren hat es sich als vorteilhaft verwiesen, wenn ein maximaler Abstand der zweiten Elastifizierungsmittel voneinander (Abstand von unmittelbar nebeneinander liegenden Elastifizierungsmiteln) an einem Absorptionskörperrand oder an einem Längsrand des Schrittabschnitts 7 bis 35 mm, insbesondere 10 bis 32 mm und weiter insbesondere 12 bis 30 mm beträgt.

Des Weiteren hat es sich als vorteilhaft erwiesen, wenn die zweiten Elastifizierungsmittel einen Auffächerungsgrad F

$$F = (A-B)/B * 100\%$$

von 50 bis 900 %, insbesondere von 100 bis 700 % und weiter insbesondere von 150 bis 550 % haben.

Dabei ist der Auffächerungsgrad F als Verhältnis der Abstandszunahme (A-B) zum minimalen Abstand (B) in Prozent definiert. Dabei sind die Größen A und B definiert als der Abstand des in Längsrichtung äußersten zweiten Elastifizierungsmittels von dem in Längsrichtung innersten zweiten Elastifizierungsmittel (also nicht der Abstand von unmittelbar nebeneinander liegenden zweiten Elastifizierungsmitteln), und zwar A als der maximale Abstand, insbesondere am Längsrand des Schrittabschnitts oder am Absorptionskörperrand, und B als der minimale Abstand insbesondere im Seitennahtbereich. Es wurde auch erkannt, dass es sich als vorteilhaft erweist, wenn der Auffächerungsgrad F der zweiten Elastifizierungsmittel im Rückenabschnitt größer ist als im Bauchabschnitt.

Aufgrund der naturbedingten Körperformen im Rückenbereich bzw. Bauchbereich eines Benutzers erweisen sich die hier thematisierten Probleme typischerweise im Rücken- oder Gesäßbereich als gravierender. Insofern erweist es sich als vorteilhaft, wenn der maximale Abstand der zweiten Elastifizierungsmittel voneinander an einem Absorptionskörperrand im Rückenabschnitt größer ist als im Bauchabschnitt.

In einer vorteilhaften Ausführungsform erstrekken sich die jeweiligen zweiten Fügebereiche und damit die Verstärkungsbereiche in Längsrichtung betrachtet beginnend vom schrittzugewandten Querrand des Bauchabschnitts und des Rückenabschnitts in Richtung auf die Längsenden des Schrittabschnitts, und zwar zumindest bis zu der Länge, dass die von den Seitennahtbereichen in Richtung auf die Längsmittelachse des Inkontinenzartikels bogenförmig verlaufenden, und insbesondere mit zunehmenden Abstand auffächernden und an den Längsrand des Schrittabschnitts mündenden zweiten Elastifizierungsmittel damit erfasst werden. Weiter vorteilhafterweise erstrecken sich die jeweiligen zweiten Fügebereiche und damit die Verstärkungsbereiche beginnend vom schrittzugewandten Querrand des Bauchabschnitts und des Rückenabschnitts in Längsrichtung betrachtet zumindest bis zu der Länge, welche beim Anlegen einer gedachten horizontalen Linie fruchtend mit der Höhe des Seitennahtbereichs übereinstimmt, von welchem aus die bogenförmig verlaufenden, und insbesondere sich auffächernden zweiten Elastifizierungsmittel beginnen. Wie eingangs beschrieben, wirkt sich die Versteifung mittels des durch den zweiten Fügebereich bereitgestellten Verstärkungsbereiches vorteilhaft auf die Passform aus.

Es wäre durchaus denkbar, dass die zweiten Elastifizierungsmittel von dem einen Seitennahtbereich zu dem anderen Seitennahtbereich durchgehend verlaufen, was insbesondere die Einbringung in einem kontinuierlichen Herstellungsverfahen im Vergleich zu einem "cut-and-place-Prozess vereinfacht. Infolge der Überdeckung des Schrittabschnitts mit dem Bauchabschnitt und mit dem Rückenabschnitt kann es je nach Gestaltung auch zu einer Überlappung oder Überdeckung des materialreichen Absorptionskörpers mit dem Bauchabschnitt und/oder dem Brückenabschnitt kommen und somit auch mit demjenigen schrittseitigen und den Beinöffnungen zugewandten Bereich des Bauchabschnitts und des Rückenabschnitts, in dem die zweiten Elastifizierungsmittel verlaufen. Der materialreiche Absorptionskörper behindert dabei üblicherweise eine elastische Dehnbarkeit der Chassismaterialien. Außerdem ist es nicht unbedingt vorteilhaft, wenn der materialreiche Absorptionskörper mit zusätzlichen Spannungskräften beaufschlagt wird. Deshalb kann es sich als vorteilhaft erweisen, wenn die zweiten Elastifizierungsmittel in einem Obertappungsbereich mit dem Absorptionskörper des Schrittabschnitts hinsichtlich ihrer elastischen Eigenschaften deaktiviert sind. Diese Deaktivierung kann beispielsweise durch eine Anzahl von Trennschnitten durch die zweiten Elastifizierungsmittel im Bereich der Überdeckung mit dem Absorptionskörper realisiert werden, wobei auch andere Trennverfahren, wie z. Bsp. mittels Ultraschallschweißen oder Laser denkbar sind.

Es sei erwähnt, dass auch die ersten Elastifizierungmittel im Bereich einer Überdeckung mit dem Absorptionskörper hinsichtlich ihrer elastischen Eigenschaften deaktiviert sein können.

Hinsichtlich der Gesamtabmessungen des Inkontinenzartikels erweist es sich als vorteilhaft, wenn der Abstand (C) des schrittzugewandten innersten zweiten Elastifizierungsmittels des Bauchabschnitts von dem entsprechenden schrittzugewandten innersten zweiten Elastifizierungsmittel des Rückenabschnitts 250 bis 420 mm beträgt.

Der Abstand der innersten, schrittzugewandten zweiten Elastifizierungsmittel von der die Beinöffnungen begrenzenden Randkontur des schrittseitigen und den Beinöffnungen zugewandten Bereichs des Bauchabschnitts und des Rückenabschnitts beträgt vorzugsweise 2 - 40 mm, weiter vorzugsweise 3 - 30 mm, insbesondere vorzugsweise 4 - 15 mm.

Als erste und/oder zweite Elastifizierungsmittel werden vorzugsweise faden- oder bandförmige Elastifizierungsmittel, wie Gummi- oder Polyetherpolyurethanoder Polyesterpolyurethanfäden, vorzugsweise Elastikfäden wie Lycra®-, Creora {R}- oder Spandex®-Fäden eingesetzt. Die ersten und/oder zweiten Elastifizierungsmittel haben vorzugsweise eine Stärke von 300 - 1500 dtex, insbesondere von 500 - 900 dtex, weiter insbesondere von 500 - 600 dtex. Die ersten und/oder zweiten Elastifizierungsmittel werden vorzugsweise unter einer Vorspannung von 1,5 - 6,0, insbesondere von 2,5 - 5,0 an derr chassisbildenden Hüllmaterialien des Bauchabschnitts und Rückenabschnitts fixiert. Die Vorspannung ist dabei definiert als Faktor des Dehnungsgrades gegenüber dem ungedehnten/relaxierten Zustand des Elastifizierungsmittels.

Dessen ungeachtet erweist es sich als vorteilhaft, wenn der Bauchabschnitt und der Rückenabschnitt zumindest au Berhalb des Absorptionskörpers über die Längsrichtung im Wesentlichen durchgehend flächenhaft querelastifiziert sind, wobei auch solchenfalls die vorteilhaften Spannungsverhältnisse einzuhalten bzw. zu realisieren sind.

Der Absorptionskörper umfasst Körperflüssigkeiten absorbierende Materialien wie natürliche oder synthetische Fasern, insbesondere Zellulosefasern, vorzugsweise in Form von Zellstofffluff. Vorzugsweise umfasst der Absorptionskern außerdem superabsorbierende Materialien (SAP), insbesondere auf Basis oberflächenvernetzter, teilneutralisierter Polyacrylate.

Der Schrittabschnitt bzw. die Längsränder des Schrittabschnitts, welche die Beinöffnungen begrenzen, sind vorteilhafterweise bogenförmig konturiert ausgebildet.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Inkontinenzartikels. In der Zeichnung zeigt:

Figur 1     eine Draufsicht auf einen erfindungsgemäßen Inkontinenzartikel, wobei ein Bauchabschnitt, ein Rückenabschnitt und ein die beiden verbindender Schrittabschnitt des Inkontinenzartikels noch nicht zur Bildung einer Höschenform verbunden, sondern in flachgelegtem und ausgedehntem Zustand dargestellt sind;

Figur 2     eine schnittansicht (schematisch) entlang einer Quermittelachse des Schrittabschnitts und daran gebundenen Rükkenabschnitts mit Schnittebene **II-II** in Figur 1;

Figur 3     eine ausschnittsweise Darstellung eines zweiten Fügebereichs des Inkontinenzartikels nach Figur 1;

Figur 4     eine Schnittansicht (schematisch) des zweiten Fügebereichs mit Schnittebene IV-IV in Figur 3;

Figur 5     eine Schnittansicht (schematisch) entlang einer Quermittelachse des Schrittabschnitts mit Schnittebene V-V in Figur 1;

Figur 6     eine perspektivische Ansicht (schematisch) des an einen Benutzer angelegten Inkontinenzartikels nach Figur 1;

Figur 7     eine ausschnittsweise Darstellung des Inkontinenzartikels nach Figur 1;

Figuren 8,9     verdeutlichen beispielhaft die Bestimmung von Rückstellkräften im Bauchabschnitt bzw. Rükkenabschnitt des Inkontinenzartikels;

Figur 10     eine Draufsicht auf einen schematisch dargestellten Inkontinenzartikel im flachgelegten und ausgedehnten Zustand, wobei der Schrittabschnitt zunächst nur mit einem Bauchabschnitt verbunden dargestellt ist.

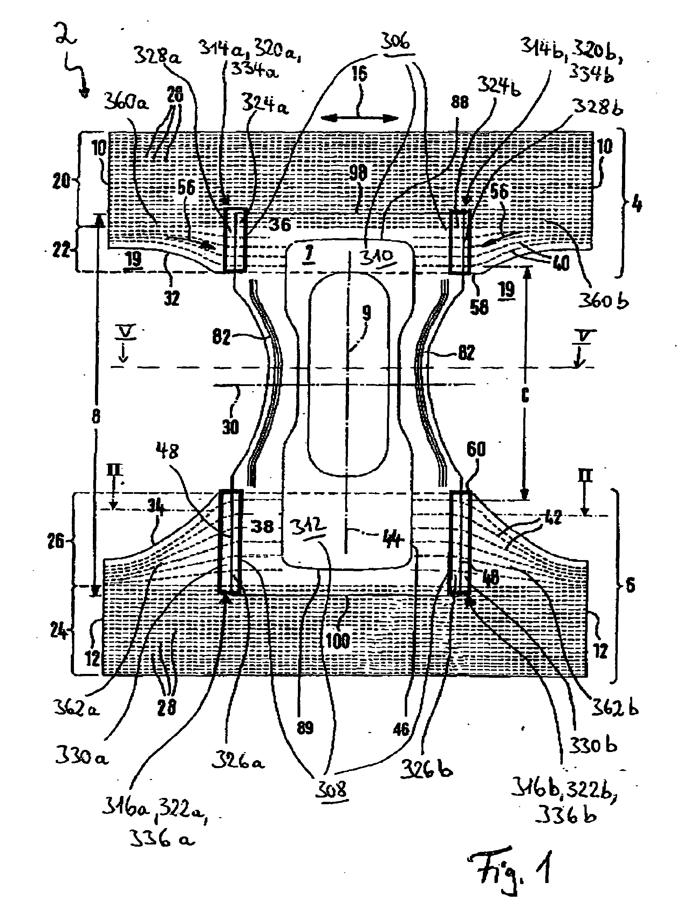

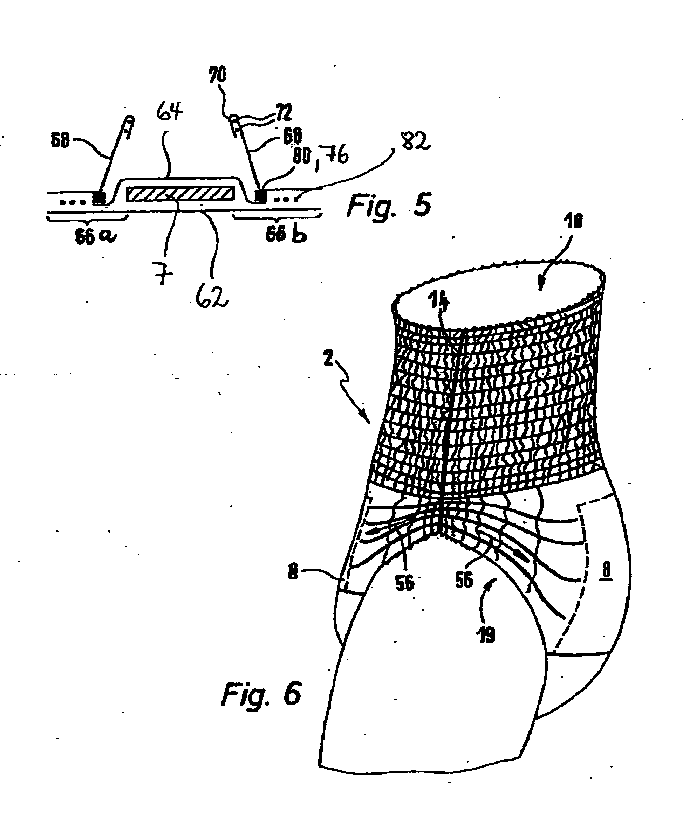

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 mit einem wesentlichen Flächenanteil mit dem Bauchabschnitt 4 einerseits und dem Rükkenabschnitt 6 andererseits überlappt und im Überlappungsbereich herstellerseitig unlösbar verbunden ist Wie aus Figur 1 ersichtlich, führt dies zu einer H-förmigen Grundstruktur des Inkontinenzartikels mit einer Längsrichtung 9. Die in Figur 1 dargestellten miteinander gefügten Bestandteile werden dann zur Bildung der in Figur 6 schematisch dargestellten Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 (Figur 6) ausgebindet werden. In diesem herstellerseitig gefertigten höschenförmigen Zustand des Inkontinenzartikels erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 und Beinöffnungen 19, durch welche hindurch der Benutzer den Inkontinenzartikel wie ein Höschen anlegt.

Der Bauchabschnitt 4 lässt sich in einen hüftseitigen Bereich 20 und in einen schrittseitigen und den Beinöffnungen zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen zugewandten Bereich 26.

In dem hüftseitigen Bereich 20 des Bauchabschnitts 4 und in dem hüftseitigen Bereich 24 des Rükkenabschnitts 6 sind erste Elastifizierungsmittel 28 vorgesehen, bei denen es sich insbesondere um fadenförmige Elastifizierungsmittel, wie Lycra®-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28 erstrecken sich in Quer- oder Hüftumfangsrichtung 16 von einem Seitennahtbereich 14 zum anderen.

Der schrittseitige und den Beinöffnungen 19 zugewandte Abschnitt 22 des Bauchabschnitts 4 bzw. 26 des Rückenabschnitts 6 haben eine von der Quer- oder Hüftumfangsrichtung 16 abweichende in Richtung auf eine Quermittelachse 30 des Schrittabschnitts 8 zulaufende Randkuntur 32 bzw. 34. Diese Randkontur 32, 34 ist auch in der Darstellung nach Figur 1 bogenförmig und daher zur Begrenzung der Beinöffnungen 19 geeignet. Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist.

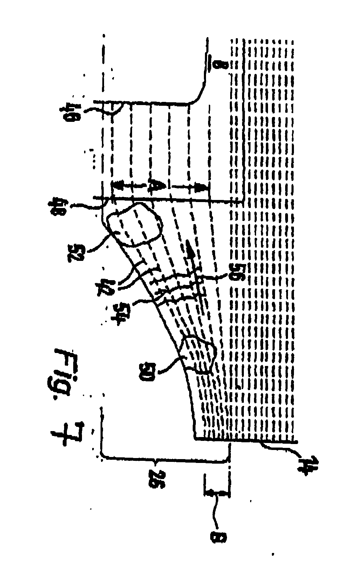

Der jeweiligen schrittseitige und den Beinöffnungen 19 zugewandte Bereich 22, 26 des Bauchabschnitts 4 bzw. des Rückenabschnitts 6 ist ebenfalls elastifiziert ausgebindet. Dort sind zweite Elastifizierungsmittel 40 bzw- 42 vorgesehen. Die zweiten Elastfizierungsmittel 40, 42 erstrecken sich jeweils ausgehend von den Seitennahtbereichen 14 in Richtung auf eine Längsmittelachse 44 des Inkontinenzartikels. Wie aus den Figuren 1 und 7 zu erkennen ist, fächern die zweiten Elastizierungsmittel 40, 42 in Richtung auf die Längsmittelachse 44 auf. Dies bedeutet, dass der Abstand zwischen ihnen in Richtung auf die Längsmittelachse 44 zunimmt. Diese Auffächerung der zweiten Elastifizierungsmittel 40 bzw. 42 lässt sich anhand Figur 7 auch quantitativ näher bezeichnen. Beispielsweise haben die in Figur 7 dargestellten zweiten Elastifizierungsmittel 42 des Rückenabschnitts 6 in den Seitennahtbereichen 14 einen minimalen Abstand von 3 bis 8 mm voneinander (Abstand von unmittelbar nebeneinander liegenden Elastifizierungsmitteln) und an einem Absorptionskörperrand 46 oder einem Längsrand 48 des Schrittabschnitts 8 einen maximalen Abstand (Abstand von unmittelbar nebeneinander liegenden Elastifizierungsmitteln) von 7 bis 35 mm voneinander. Es lässt sich anhand der Figur 7 auch ein Auffächerungsgrad F wie folgt definieren:

$$F = (A-B)/B * 100\%.$$

Dieser Auffächerungsgrad kann vorteilhafterweise zwischen 50 und 900 %, insbesondere zwischen 100 und 700 % und weiter insbesondere zwischen 150 bis 560 % liegen. Er ist im Rückenabschnitt 6 vorteilhafterweise größer als im Bauchabschnitt 4. Dabei sind die Größen A und B definiert als der Abstand des in Längsrichtung 9 äu Bersten zweiten Elastifizierungsmittels 40, 42 von dem in Längsrichtung 9 innersten zweiten Elastifizierungsmittel 40, 42 (also nicht der Abstand von unmittelbar nebeneinander (liegenden Elastifizierungsmitteln), und zwar A als der maximale Abstand, insbesondere am Längsrand 48 des Schrittabschnitts 8, und B als der minimale Abstand, insbesondere im Seitennahtbereich 14 (vgl. Figur 7).

Sofern der Auffächerungsgrad bei den zweiten Elastifizierungsmitteln 40, 42 hinreichend groß gewählt wird, so lässt sich auf diese Weise eine abnehmende Rückstellkraft innerhalb des schrittseitigen und den Beinöffnungen 19 zugewandten Bereichs 22 beziehungsweise 26 in Richtung 56 auf den Schrittabschnitt 8 realisieren, sofern dafür Sorge getragen wird, dass in Folge des von der Hüft- oder Querrichtung 16 abgewandten bogenförmigen Verlaufs der zweiten Elastifizierungsmittel 40, 42 sich keine zu starke Erhöhung der Vorspannung in Folge des größeren Wegs dieser zweiten Elastifizierungsmittel 40, 42 ergibt. Betrachtet man dann ein näher am Seitennahtbereich 14 liegendes Gebiet 50 des betreffenden schrittseitigen Bereichs 22 beziehungsweise 26 mit einem näher am Schrittabschnitt 8 liegenden Gebiet 52, so ist die Rückstellkraft, die sich bei flächenhafter Dehnung des Gebiets 52 (Dehnung in Richtung der Elastifizierungsmittel 42) einstellt geringer als diejenige Rückstellkraft, die sich bei Dehnung des Gebiets 50 einstellt. Dies führt in vorteilhafter Weise weiter dazu, dass in Folge der geringeren elastischen Kräfte, welche im beispielhaft dargestellten Fall durch die zweiten Elastifizierungsmittel 40, 42 ausgeübt werden, die Chassismaterialien des Bauchabschnitts 4 und des Rückenabschnitts 6 weniger stark gerafft werden, so dass sich auch eine geringere Anzahl von Falten/Rüschen 54 ergibt, und zwar ausgehend vom jeweiligen Seitennahtbereich 14 in Richtung auf den Schrittabschnitt 8. Dadurch, dass die sich bei flächenhafter Dehnung des Bauchabschnitts in dem schrittseitigen und den Beinöffnungen zugewandten Bereich 22 des Bauchabschnitts 4 beziehungsweise 26 des Rückenabschnitts 6 sich einstellenden Rückstellkräfte in Richtung des Pfeils 56, also generell vom Seitennahtbereich 14 in Richtung auf den Schrittabschnitt 8 abnehmen, wird eine erhebliche Verbesserung des Tragekomforts erzielt, weil gerade in diesen Bereichen - wie festgestellt wurde - sich elastisch dehnbare Materialien als besonders problematisch erweisen, weil diese Materialien der Physiognomie der menschlichen Körperform entsprechend in diesen Bereichen besonders auf Zug und Dehnung beansprucht werden. Durch eine bewusst vorteilhaft vorgesehene Verringerung dieser Rückstellkraft, also abnehmende Rückstellkraft in Richtung des Pfeils 56, das heißt in Richtung zunehmender Annäherung an den Schrittabschnitt 8, wird hier ein zuvor nicht realisierter Freiheitsgrad geschaffen.

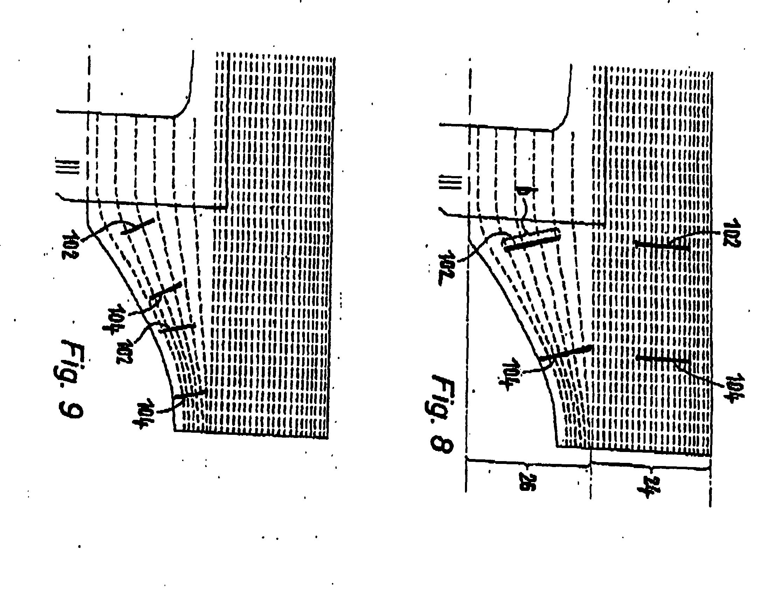

Wie eingangs ausgeführt, können Rückstellkräfte direkt am Chassis des Inkontinenzartikels bestimmt werden. Hierfür wird der betreffende Bereich des Bauchabschnitts 4 oder des Rückenabschnitts zwischen zwei Klemmbacken 102, 104 (siehe Fig. 8, 9) von definierter, gleicher Klemmbackenbreite (b) eingespannt, und es werden dann Rückstellkräfte bei einer definierten, den Gebrauchszustand simulierenden Dehnung der zu messenden Bereiche um insbesondere 30% oder 50% oder 80% der Ausgangslänge (des Klemmbackenabstandds im ungespannten Zustand) ermittelt. Die Klemmbacke 102, 104 werden dabei jeweils voneinander wegbewegt. Die Klemmbacken 102, 104 sollten möglichst viele, zumindest jedoch zwei nebeneinander angeordnete Elastifizierungsmittel 40,42 des zu messenden Bereichs fixieren, und sie sollten im Wesentlichen rechtwinklig zum Verlauf der Elastfizierungsmittel orientiert sein, so das die Dehnung zwischen den Klemmbacken 102, 104, also die Auseinanderbewegung der Klemmbacken 102, 104, im Wesentlichen in Richtung des Verlaufs der Elastifizierungsmittel erfolgt. Dies ist in Figuren 8 und 9 verwirklicht.

Bei der dargestellten bevorzugten Ausführungsform des Inkontinenzartikels 2 beträgt ein Abstand C des schrittzugewandten innersten zweiten Elastifizierungsmittels 40 des Bauchabschnitts 4 von dem entsprechenden schrittwgewandten innersten zweiten Elastifizierungsmittel 42 des Rückenabschnitts 6 zwischen 250 bis 420 mm je nach Konfektionsgröße des Inkontinenzartikels. Die zweiten Elastifizierungsmittel 40, 42 erstrecken sich im Wesentlichen bis zum schrittzugewandten Querrand 58, 60 des Bauchabschnitts 4 und des Rückenabschnitts 6. Der Abstand von Bauchabschnitt 4 und Rückenabschnitt 6 voneinander beträgt 250 - 400 mm.

Der Abstand der innersten, schrittzugewandten zweiten Elastifizierungsmittel 40, 42 von der die Beinöffnungen begrenzenden Randkontur 32, 34 des schrittseitigen und den Beinöffnungen zugewandten Bereichs 22, 26 des Bauchabschnitts 4 und des Rückenabschnitts 6 beträgt vorzugsweise 2 - 40 mm, weiter vorzugsweise 3 - 30 mm, insbesondere vorzugsweise 4 -15 mm.

Die Erstreckung des Bauchabschnitts 4 und des Rückenabschnitts 6 im Seitennahtbereich 14 in Längsrichtung 9 beträgt vorteilhafterweise zwischen 100 und 220 mm. Die Erstreckung des Schrittabschnitts 8 in Querrichtung 16 beträgt vorteilhafterweise 200 bis 350 mm.

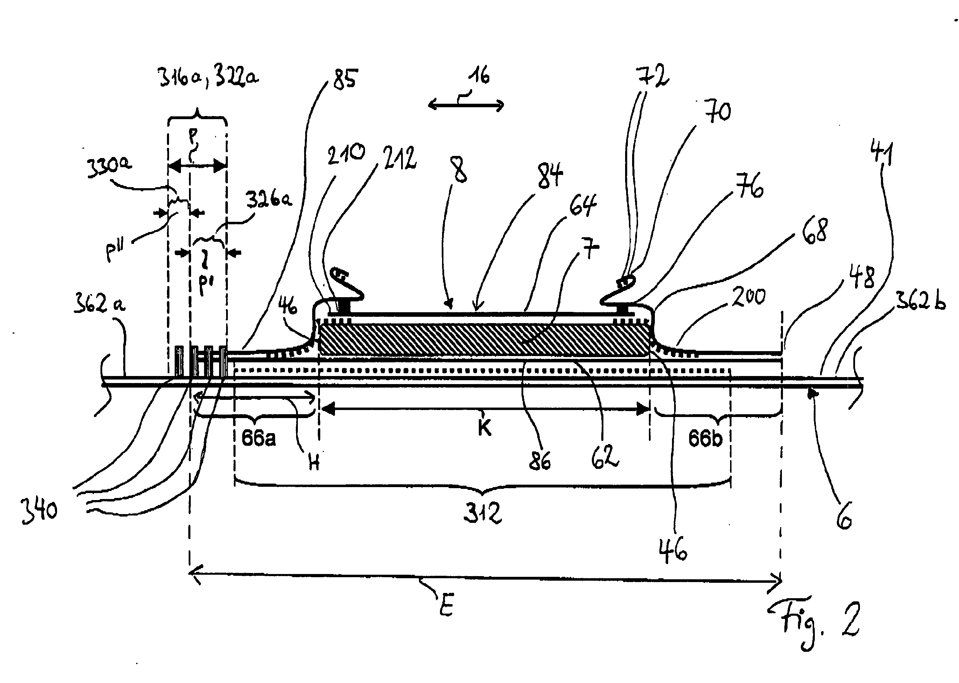

Wie in Figur 2 ersichtlich, welche schematisch eine Schnittansicht entlang der Schnittebene II - II aus der Figur 1 darstellt, umfasst der Schrittabschnitt 8 ein flüssigkeitsundunchlässiges Backsheet-Material 62, das insbesondere von einem atmungsaktiven, jedoch flüssigkeitsdichten Folienmaterial gebildet sein kann, und ein, vorzugsweise auf Vliesbasis hergestelltes Deckblatt-Material 84, welches ein Verbund aus einem auf Vliesbasis hergestelltem Topsheet-Material 64 und aus beidseits angeordneten Barrieremitteln 68 ist. Zwischen dem Backsheet-Material und dem Topsheet-Material ist, wie aus der Figur 2 ersichtlich, der Absorptionskörper 7 angeordnet. Der Absorptionskörper 7 weist Längsränder 46 auf. Im beispielhaft dargestellten Fall bildet das Backsheet-Material 62 in Querrichtung 16 beidseits der Längsränder 46 jeweils einen Überhang 66a, 66b. Das Topsheet 64 überragt den Absorptionskörper 7 in Querrichtung nur verhältnismäßig geringfügig; jedoch ist beidseits des Absorptionskörpers 7 in Längsrichtung 9 verlaufend jeweils ein aufstehendes Barrieremittel 68 vorgesehen, welches typischerweise als aufstehendes Cuffelement oder Bündchenelement bezeichnet wird und vorzugsweise aus einem hydrophoben, insbesondere flüssigkeitsundurchlässigen Vliesstoffmaterial gebildet ist, welches sich in Querrichtung 16 vorzugsweise bis zu den seitlichen Längsrändern 48 des Schrittabschnitts 8 erstreckt. Das Barrieremittel 68 ist dabei in Fügestellen 76 an den Längsrändern 210 bzw. den Längsrandbereichen 212 des Topsheet-Materials 64 angefügt. Die distalen Enden 70 der Barrieremittel 68 sind mit weiteren Elastifizierungsmitteln 72 versehen, welche die Barrieremittel 68 im Gebrauch des Inkontinenzartikels gegen die Hautoberfläche des Benutzers anheben, so wie dies in Figur 5, eine schematische Schnittansicht entlang der Schnittebene IV - IV aus der Figur 1, dargestellt ist. Die in Längsrichtung 9 angeordneten Fügestellen 76 bilden die durchgehend erstreckte Cuffsockellinie 80.

Es erweist sich hier als besonders vorteilhaft, wenn der erwähnte Überhang 66a, 66b des Backsheet-Materials 62 und/oder des Deckblatt-Materials 84 beidseits der Längsränder 46 des Absorptionskörpers 7, also in der Summe, wenigstens 25 % bezogen auf die größte Breite E des Schrittabschnitts 8 beträgt. Auf diese Weise ist nämlich in Querrichtung 16 Raum für die Anordnung entlang der Beinöffnungen 19 erstreckter Beinelastifizierungsmittel 82. Es erweist sich nämlich als vorteilhaft, wenn die Beinelastifizierungsmittel 82 mit einem gewissen Abstand vom materialreichen und damit eher biegesteifen Absorptionskörper 7 verlaufen, um einerseits keine zusätzlichen Dehnungs- oder Verwindungskräfte auf den Absorptionskörper auszuüben, was dessen Absorptionsverhalten nachteilig beeinflussen könnte, und um andererseits einen vom Absorptionskorper weitgehend unbeeinflussten flüssigkeitsdichten Beinabschluss zu realisieren. Es erweist sich im dargestellten Fall als besonders vorteilhaft, dass diese Beinelastifizierungsmittel 82 in Längsrichtung 9 mit einem deutlichen Abstand von insbesondere wenigstens 10 mm, vorzugsweise wenigstens 20 mm vor den zweiten Elastifizierungsmitteln 40 und 42 des Bauchabschnitts 4 beziehungsweise des Rückenabschnitts 6 enden. Vorzugsweise enden diese Beinelastifizierungsmittel 82 in Längsrichtung 9 vor dem Bauchabschnitt 4 und dem Rückenabschnitt 6. Dies ist deshalb vorteilhaft und wesentlich, weil die Beinelastifizierungsmittel 82 solchenfalls das Spannungsverhalten des Bauchabschnitts 4 und des Rückenabschnitts 6 wenig oder gar nicht beeinflussen. Es wurde nämlich erkannt, dass es sich im Hinblick auf das vorteilhaft zu erreichende Ziel der Verbesserung des Tragekomforts gerade in dem schrittseitigen und den Beinöffnungen 19 zugewandten Bereich 22 und 26 des Bauchabschnitts 4 und des Rückenabschnitts 6 als negativ erweist, wenn dort die üblicherweise mit großer Vorspannung und entsprechend großer Rückstellkraft ausgebildeten Beinelastifizierungsmittel 82 zusätzlich verlaufen.

Wie aus Figur 2 in Verbindung mit Figur 1 ersichtlich ist, ist bei dem Schrittabschnitt 8 ein in Querrichtung 16 verhältnismäßig großer Überhang 66a, 66b beidseits der Längsränder 46 des Absorptionskörpers 7 realisiert, und zwar insbesondere auch an dem Bauchabschnitt 4 beziehungsweise dem Rückenabschnitt 6 zugewandten Bereichen des Schrittabschnitts 8. Hierdurch wird - worauf bereits hingewiesen wurde - ein verhältnismäßig großer Überlappungsbereich 36, 38 des Schritfabschnitts 8 mit dem Bauchabschnitt 4 und mit dem Rückenabschnitt 6 realisiert. Nach einer bevorzugten Ausführungsvariante umfasst der Überlappungsbereich 36 von Schrittabschnitt 8 mit dem Bauchabschnitt 4 wenigstens 12% der Fläche des Bauchabschnitts 4, und der Überlappungsbereich 38 von Schrittabschnitt 8 mit dem Rückenabschnitt 6 umfasst wenigstens 20% der Fläche des Rückenabschnitts 6. Dies erweist sich als vorteilhaft, da solchenfalls eine sichere Fixierung des Schrittabschnitts 8 an dem Bauchabschnitt 4 beziehungsweise an dem Rückenabschnitt 6 erreicht werden kann.

Figur 1 zeigt einen Inkontinenzartikel 2 mit einer H-förmigen Grundstruktur in seinem ebenen ausgebreiteten Zustand.

Wie in Figur 1 ersichtlich ist, weist der Inkontinenzartikel 2 die efindungsgemäßen ersten Fügebereiche 310, 312 und zweiten Fügebereiche 314a, 314b, 316a, 316b auf, welche im vorderen und im hinteren Überlappungsbereich 36, 38 angeordnet sind und damit einen vorderen und einen hinteren Verbindungsbereich 306, 308 bereitstellen, in denen der Schrittabschnitt 8 an den Bauchabschnitt 4 bzw. an den Rückenabschnitt 6 unlösbar angefügt ist.

Zusammen mit der Figur 2, welche eine schematische Schnittansicht entlang der Ebene II - II aus Figur 1 darstellt (dabei wurde aber nur linkerhand der zweite Fügebereich schematisch eingezeichnet), wird ersichtlich, dass der zweite Fügebereich 314a, 314b, 316a, 316b dabei in einem den jeweiligen Längsrand 48 des Schrittabschnitts 8 überbrückenden Bereich 320a, 320b, 322a, 322b angeordnet ist. Damit wird durch den zweiten Fügebereich 314a, 314b, 316a, 316b mit den darin angeordneten Fügemitteln 340 jeweils sowohl ein Teilbereich 324a, 324b, 326a, 326b des Überhangs 66a, 66b als auch ein an den Längsrand 48 angrenzender Teilbereich 328a, 328b, 330a, 330b des Bauchabschnitts 4 oder Rückenabschnitts 6 überfangen.

Diese zweiten Fügebereiche 314a, 314b, 316a, 316b bilden jeweils einen Verstärkungsbereich 334a, 334b, 336a, 336b aus.

Die zweiten Fügebereiche 314a, 314b, 316a, 316b und damit die Verstärkungsbereiche 334a, 334b, 336a, 336b erstrecken sich in Längsrichtung 9 betrachtet beginnend vom schrittzugewandten Querrand 58, 60 des Bauchabschnitts 4 und des Rückenabschnitts 6 kontinuierlich in Richtung auf die Längsenden des Schrittabschnitts, vorzugsweise bis mindestens zu einem jeweiligen Längsende 98, 100 des Schrittabschnitts 8.

Die zweiten Fügebereiche 314a, 314b, 316a, 316b weisen eine Gesamtbreite P von vorzugsweise 5 - 60 mm, insbesondere von 10 - 50 mm, insbesondere von 10 - 40 mm, weiter insbesondere 10 - 30 mm auf.

In dem vom zweiten Fügebereich 314a, 314b, 316a, 316b überfangenden Teilbereich 324a, 324b, 326a, 326b des jeweiligen Überhangs 66a, 66b weist der zweite Fügebereich eine Breite P' von vorzugsweise größer 1 mm, weiter vorzugsweise größer 2 mm, weiter vorzugsweise größer 5 mm und aber vorzugsweise kleiner 60 mm, weiter vorzugsweise kleiner 50 mm, weiter vorzugsweise kleiner 40 mm, weiter vorzugsweise kleiner 30 mm, weiter vorzugsweise kleiner 20 mm, insbesondere vorzugsweise von 10 mm auf.

Der zweite Fügebereich 314a, 314b, 316a, 316b überfängt den jeweiligen Teilbereich 324a, 324b, 326a, 326b des Überhangs 66a, 66b in Querrichtung derart, dass der Anteil Anteil P7 H des durch den zweiten Fügebereich 314a, 314b, 318a, 316b überfangenen Teilbereiches 324a, 324b, 326a, 326b des jeweiligen Überhangs 66a, 66b mit der Breite P' bezogen auf den jeweiligen Überhang 66a, 66b mit der Breite H vorzugsweise mindestens 0,01, insbesondere mindestens 0,04, weiter insbesondere vorzugsweise mindestens 0,07, weiter insbesondere mindestens 0,10, und aber vorzugsweise höchstens 0,90, weiter insbesondere höchstens 0,80, weiter insbesondere höchstens 0,70, weiter insbesondere höchstens 0,60, weiter insbesondere höchstens 0,50, weiter insbesondere höchstens 0,40 beträgt.

In dem vom zweiten Fügebereich 314a, 314b, 316a, 316b überfangenden Teilbereich 328a, 328b, 330a, 330b des Bauchabschnitts 4 oder Rückenabschnitts 6 weist der zweite Fügebereich eine Breite P" vorzugsweise größer 1 mm, weiter vorzugsweise größer 2 mm, weiter vorzugsweise größer 5 mm und aber vorzugsweise kleiner 60 mm, weiter vorzugsweise kleiner 50 mm, weiter vorzugsweise kleiner 40 mm, weiter vorzugsweise kleiner 30 mm, weiter vorzugsweise kleiner 20 mm, insbesondere vorzugsweise von 10 mm auf.

Der zweite Fügebereich 314a, 314b. 316a, 316b überfängt den jeweiligen Teilbereich 328a, 328b, 330a, 330b des Bauchabschnitts 4 und des Rückenabschnitts 6 in Querrichtung 16 derart, dass der Anteil P"/ H des jeweiligen durch den zweiten Fügebereich 314a, 314b, 316a, 316b überfangenen Teilbereiches 328a, 328b, 330a, 330b des Bauchabschnitts und Rückenabschnitts mit der Breite P" bezogen auf den jeweiligen Überhang 66a, 66b mit der Breite H vorzugsweise mindestens 0,01, insbesondere mindestens 0,04, weiter insbesondere mindestens 0,07, weiter insbesondere mindestens 0,10 und aber vorzugsweise höchstens 0,90, insbesondere höchstens 0,80, weiter insbesondere höchstens 0,70, weiter insbesondere höchstens 0,60, weiter insbesondere höchstens 0,50 und weiter insbesondere höchstens 0,40 beträgt.

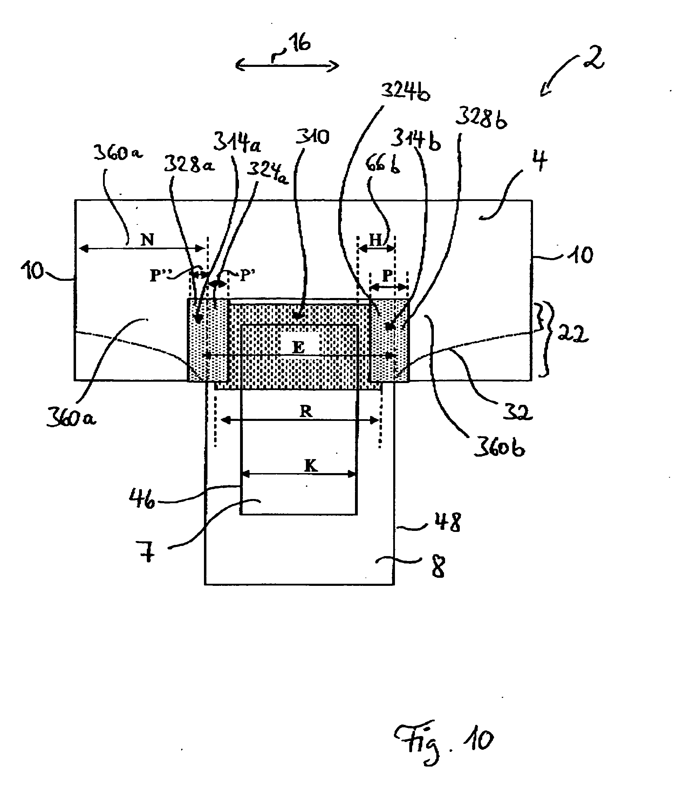

Wie voranstehend erläutert, überfängt der zweite Fügebereich 314a, 314b, 316a, 316b einen angrenzenden Teilbereich 328a, 328b, 330a, 330b des Bauchabschnitts und des Rückenabschnitts. Zwischen dem Längsrand 48 des Schrittabschnitts 8 und dem Längsrandabschnitt 10, 12 des Bauchabschnitts 4 und Rückenabschnitts 6 sind die jeweiligen Seitenbereiche 360a, 360b, 362a, 362b. Der jeweilige Seitenbereich weist eine Breite N auf. Wie in Figur 10 weiter schematisch dargestellt, wird durch den zweiten Fügebereich 314a, 314b (bzw. 316a, 316b) mit der anteiligen Breite P" der jeweilige Seitenbereich 360a, 360b (bzw. 362a, 362b) des Bauchabschnitts bzw. des Rückenabschnitts mit der Breite N überfangen. In Figur 10 ist der Bauchabschnitt bzw. Rückenabschnitt als rechteckförmiges Panel dargestellt. Der dem Schritt zugewandte Bereich 22 kann aber, so wie in Figur 1 dargestellt und auch wie in Figur 10 gestrichelt eingezeichnet, eine bogenförmige Randkontur 32 einnehmen.

Der Anteil P"/N des jeweiligen durch den zweiten Fügebereich 314a, 314b, 316a, 316b überfangenen Teilbereiches 328a, 328b, 330a, 330b des Bauchabschnitts und/oder Rückenabschnitts mit der Breite P" bezogen den jeweiligen Seitenbereich 360a, 360b, 362a, 362b des Bauchabschnitts und/oder Rückenabschnitts mit der Breite N beträgt vorzugsweise mindestens 0,01, insbesondere mindestens 0,015, weiter insbesondere mindestens 0,020, und vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10.

In einer vorteilhaften Weise beträgt der Anteil P'/N des jeweiligen durch den zweiten Fügebereich 314a, 314b, 316a, 316b überfangenen Teilbereiches 324a, 324b, 326a, 326b des jeweiligen Überhang 66a, 66b mit der Breite P' bezogen den jeweiligen Seitenbereich 360a, 360b, 362a, 362b des Bauchabschnitts und/oder Rückenabschnitts mit der Breite N vorzugsweise mindestens 0,01, insbesondere mindestens 0,015, weiter insbesondere mindestens 0,020 und vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10.

Die in dem zweiten Fügebereich 314a, 314b, 316a, 316b (Figur 2) angeordneten Fügemittel 340 sind Schweißstellen, vorzugsweise Ultraschallschweißstellen, welche innerhalb des zweiten Fügebereiches nicht vollflächig angeordnet sind.

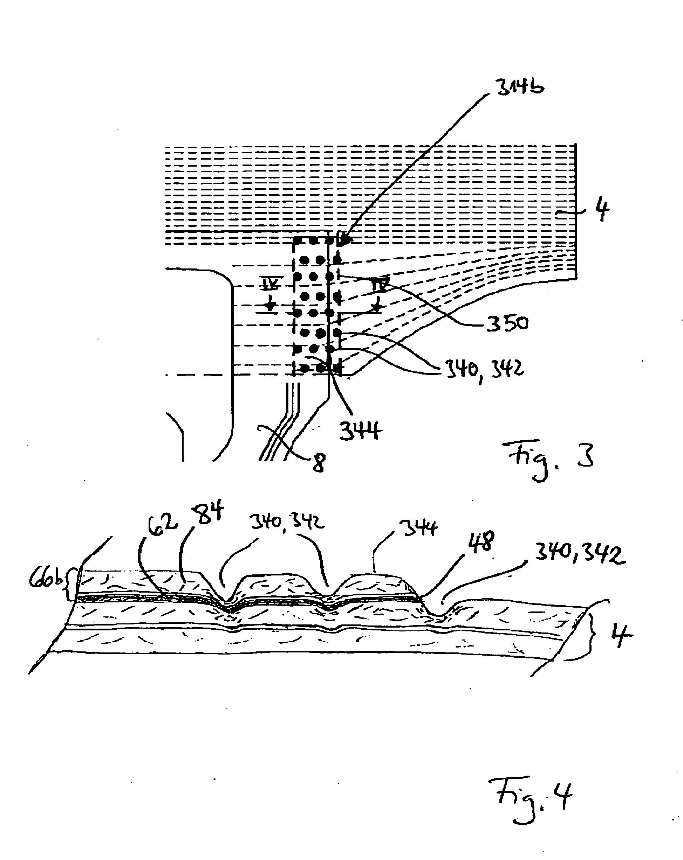

Wie weiter in Figur 3 dargestellt (hier ein Ausschnitt des Inkontinenzartikels mit der Bindung des Schnittabschnitts 8 an den Bauchabschnitt 4), sind die Fügemittel 340 punktförmig angeordnet. Die Fügemittel 340 in Form von Ultraschallschweißstellen bilden jeweils eine Fügestelle (also einen gebundenen Bereich) 342, welche von der nächsten Fügestelle durch einen ungebundene Bereich 344 beabstandet ist. Zur Bestimmung der flächenhaften Erstreckung eines zweiten Fügebereiches (hier 314b) werden die jeweils äußersten Fügemittel 340 bzw. Fügestellen 342 mittels einer imaginären Linie 350 verbunden.

Die im Punktmuster angeordneten Fügemiftel 340 bilden in der vom zweiten Fügebereich 314b überfangenen Gesamtfläche gebundene Flächen (Fügestellen), welche in Summe vorzugsweise mindestens 1.5%, insbesondere mindestens 2,0%, weiter insbesondere mindestens 2,3%, weiter insbesondere mindestens 2,5 % und vorzugsweise höchstens 20,0%, insbesondere höchstens 15,0%, weiter insbesondere höchstens 10,0%, weiter insbesondere höchstens 8,0%, weiter insbesondere höchstens 7,0%, weiter insbesondere höchstens 6,0% bezogen auf die vom zweiten Fügebereich überfangene Gesamtfläche einnehmen. Die einzelnen Fügemittel weisen einen Durchmesser vorzugsweise von mindestens 0,2 mm, insbesondere von mindestens 0,3 mm, weiter insbesondere von mindestens 0,4 mm und vorzugsweise von höchstens 2,5 mm, insbesondere höchstens 2,0 mm, weiter insbesondere höchstens 1,5 mm, weiter insbesondere höchstens 1,2 mm, insbesondere höchstens 1,0 mm auf. Die benachbarten einzelnen in dem Punktmuster vorhandenen Fügemittel 340 haben einen Abstand voneinander von vorzugsweise 1 - 10 mm, insbesondere von 1 - 8 mm, weiter insbesondere von 1 - 6 mm, weiter insbesondere von 1 - 5 mm, weiter insbesondere von 1,5 - 4,5 mm, weiter insbesondere von 2 - 4 mm.

Figur 4 stellt eine Schnittansicht des zweiten Fügebereiches 314 a entlang der Schnittebene IV-IV aus der Figur 3 dar.

Der Überhang 66b des Schrittabschnitts 8 bestehend aus einem Deckblatt-Material 84 aus Vlies und einem Backsheet 62 aus Folie ist mittels durch die Fügemittel 340 erzeugten Fügestellen 342 an den darunter liegenden Bauchabschnitt 4 gebunden.

Wie in Figur 2 dargestellt, verläuft der erste Fügebereich 312 unterhalb des Absorptionskörpers 7 mit der Breite K, erstreckt sich also auch über die Kontur, also den jeweiligen Längsrand 46 des Absorptionskörpers 7 hinaus, jedoch nicht außerhalb der Kontur des Schrittabschnitts 8, sondern endet innerhalb der Längsränder 48 des Schrittabschnitts 8. Der Schrittabschnitt 8 ist dabei vorzugsweise mittels eines vollflächigen Kleberauftrags mit dem Rückenabschnitt 6 verbunden.

Das Deckblatt-Material ist, wie im vorliegende Beispiel, ein Verbund aus einem Topsheet 64 und beidseits an den Längsrändern 210 bzw. den Längsrandbereichen 212 des Topsheets 64 angefügten Barrieremitteln 68. Das Deckblatt-Material ist dabei mittels eines Klebers 200, insbesondere eines Hotmeltklebers, an den Absorptionskörper und den Seitenrand 46 des Absorptionskörpers 7 überlappend auch noch an einen angrenzenden Teilbereich des Backsheet-Materials 62.

Auch zwischen Backsheet-Material und Absorptionskörper und zwischen Deckblatt-Material und Absorptionskörper ist ein separates Fügemittel vorgesehen (in der Figur 2 nicht dargestellt). Diese separaten Fügemittel sind in Form eines Klebers nicht vollflächig, sondern in Form eines unterbrochenen Musters aufgetragen. Diese in Form eines Klebers vorgesehenen separaten Fügemittel sind also beispielsweise rasterförmig, streifenförmig oder als Spiralmuster aufgetragen.

Figur 10 zeigt schematisch einen noch nicht fertig gestellten Inkontinenzartikel 2 mit einem an den Schrittabschnitt 8 angefügten Bauchabschnitt 4 und eine vorteilhafte Ausführung der Anordnung des ersten Fügebereichs 310 und der jeweils zweiten Fügebereiche 314a, 314b. Wie in Figur 10 dargestellt, erstreckt sich der erste Fügebereich 310 in Querrichtung 16 mit der Breite R vorteilhalterweise über die Längsränder 46 des Absorptionskörpers 7 mit einer Breite K hinaus, aber ohne sich über die Längsränder 48 des Schrittabschnitts 8 hinauszustrecken. Der jeweils zweite Fügebereich 314a, 314b mit Breite P überbrückt jeweils den Längsrand 48 des Schrittabschnitts 8. Der Fügebereich 314a, 314b überfängt dabei jeweils einen angrenzenden Teilbereich 324a, 324b des Überhangs 66a, 66b mit der Breite H , uns zwar mit der Breite P'. Der zweite Fügebereich 314a, 314b überfängt auch jeweils einen angrenzenden Teilbereich 328a, 328b des Bauchabschnitts 4, und zwar mit der Breite P". Der erste Fügebereich 310 erstreckt sich dabei derart in Querrichtung, dass eine Überlappung mit dem jeweiligen zweiten Fügebereich 324a, 324b erhalten wird.

**Patentansprüche**

1. Inkontinenzartikel (2) in Höschenform für die Aufnahme von Körperausscheidungen mit einem vorderen Bauchabschnitt (4) und einem hinteren Rükkenabschnitt (6), die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahmereichen (14) herstellerseitig miteinander verbunden sind, und mit einem einen Absorptionskörper (7) aufweisenden Schrittabschnitt (8) mit einer Innenseite (85), einer Außenseite (86), mit Längsrändem (48) und Längsenden (98,100), der sich in einer Längsrichtung (9) zwischen dem Bauchabschnitt (4) mit einer Innenseite (41) und mit einem schrittzugewandten Querrand (58) und dem Rükkenabschnitt (6) mit einer Innenseite (41) und mit einem schritzugewandten Querrand (60) erstreckt, wobei der Schrittabschnitt (8) mit dem Bauchabschnitt (4) in einem vorderen Überlappungsbereich (36) und der Schrittabschnitt (8) mit dem Rückenabschnitt (6) in einem hinteren Überlappungsbereich (38) überlappt, wobei der Schrittabschnitt (8) mit seiner Außenseite (86) an die Innenseite (41) des Bauchabschnitts (4) in einem vorderen Verbindungsbereich (306) und der Schrittabschnitt (8) mit seiner Außenseite (86) an die Innenseite (41) des Rückenabschnitts (6) in einem hinteren Verbindungsbereich (308) unlösbar angefügt ist, wobei sowohl der Schrittabschnitt (8) als auch der Bauchabschnitt (4) und der Rükkenabschnitt (6) die Beinöffnungen (19) des Inkontinenzartikels begrenzen, wobei in dem Bauchabschnitt (4) und dem Rückenabschnitt (6) erste Elastifizierungsmittel (28) vorgesehen sind, die sich in einem Abstand voneinander und parallel zueinander in Quer- oder Hüftumfangsrichtung (16) erstrecken und so den Bauchabschnitt (4) und den Rückenabschnitt (6) flächenhaft elastifizieren, wobei in einem schrittseitigen und den Beinöffnungen (19) zugewandten Bereich (22, 26) des Bauchabschnitts (4) und des Rückenabschnitts (6) zweite Elastifizierungsmittel (40, 42) vorgesehen sind, wobei der Schrittabschnitt (8) ein flüssigkeitsundurchlässiges Backsheet-Material (62) umfasst, und wobei der Absorptionskörper (7) zwischen Backsheet-Material (62) und einem Deckblatt-Material (84) angeordnet ist und der Absorptionskörper (7) Längsränder (46) aufweist, wobei das Deckblatt-Material (84) oder das Deckblatt-Material (84) und das Backsheet-Material (62) in Querrichtung (16) einen sich jeweils außerhalb der Längsränder (46) des Absorptionskörpers (7) erstreckenden Überhang (66a, 66b) bilden, wobei die Verbindungsbereiche (306, 308) des Bauchabschnitts (4) und des Ruk-

kenabschnitts (6) jeweils einen ersten Fügebereich (310, 312) und zweite Fügebereiche (314a, 314b, 316a, 316b) umfassen, wobei der erste Fügebereich (310, 312) sich zumindest abschnittsweise unterhalb des Absorptionskörpers (7) erstreckt, und wobei die zweiten Fügebereiche (314a, 314b, 316a, 316b) in einem den jeweiligen Längsrand (48) des Schrittabschnitts (8) überbrückenden Bereich (320a, 320b, 322a, 322b) vorgesehen sind, also sowohl einen Teilbereich (324a, 324b, 326a, 326b) des Überhangs (66a, 66b) des Schrittabschnitts (8) als auch einen daran angrenzenden Teilbereich (328a, 328b, 330a, 330b) des Bauchabschnitts (4) und/oder des Rückenabschnitts (6) überfangen, und wobei ein jeweiliger zweiter Fügebereich (314a, 314b, 316a, 316b) sich in Längsrichtung (9) jeweils beginnend von einem schrittzugewandten Querrand (58, 60) des Bauchabschnitts (4) und/oder des Ruckenabschnitts (6) in Richtung auf die Längsenden (98, 100) des Schrittabschnitts (8) erstreckt, und wobei ein jeweiliger zweiter Fügebereich (314a, 314b, 316a, 316b) durch Fügemittel (340) in Form von Schweißstellen, insbesondere Ultraschallschweißstellen, thermische Schweißstellen und/oder Kalanderschweißstellen gebildet ist, so dass die zweiten Fügebereiche (314a, 314b, 316a, 316b) Verstärkungsbereiche (334a, 334b, 336a, 336b) bilden.

2. Inkontinenzartikel nach Anspruch 1, **dadurch gekennzeichnet, dass** die zweiten Fügebereiche (314a, 314b, 316a, 316b) und damit die Verstärkungsbereiche (334a, 334b, 336a, 336b) in Längsrichtung (9) betrachtet beginnend vom schrittzugewandten Querrand (58, 60) des Bauchabschnitts (4) und des Rückenabschnitts (6) sich bis mindestens zum jeweiligen Längsende (98, 100) des Schrittabschnitts (8) erstrecken.

3. Inkontinenzartikel nach einem oder mehreren der vorstehenden Ansprüche, **dadurch gekennzeichnet, dass** der Anteil P'/ H des durch einen zweiten Fügebereich (314a, 314b, 316a, 316b) überfangenen Teilbereiches (324a, 324b, 326a, 326b) des jeweiligen Überhangs (66a, 66b) mit einer Breite (P') bezogen auf den jeweiligen Überhang (66a, 66b) mit einer Breite (H) im vorderen und/oder hinteren Überlappungsbereich (36, 38) mindestens 0,01, insbesondere mindestens 0,04, weiter insbesondere mindestens 0,07, weiter insbesondere mindestens 0,10, aber vorzugsweise höchstens 0,90, insbesondere höchstens 0,80, weiter insbesondere höchstens 0,70, weiter insbesondere höchstens 0.60, weiter insbesondere höchstens 0,50, weiter insbesondere höchstens 0,40 beträgt.

4. Inkontinenzartikel nach einem oder mehreren der vorstehenden Ansprüche, **dadurch gekennzeichnet, dass** der Anteil P''/ H des jeweiligen durch einen zweiten Fügebereich (314a, 314b, 316a, 316b) überfangenen Teilbereiches (328a, 328b, 330a, 330b) des Bauchabschnitts (4) und/oder des Rückenabschnitts (6) mit einer Breite (P") bezogen auf den jeweiligen Überhang (66a, 66b) mit einer Breite (H) mindestens 0,01, insbesondere mindestens 0,04, weiter insbesondere mindestens 0.07, weiter insbesondere mindestens 0,10, aber vorzugsweise höchstens 0,90, insbesondere höchstens 0,80, weiter insbesondere höchstens 0,70, weiter insbesondere höchstens 0,60, weiter insbesondere höchstens 0,50, weiter insbesondere höchstens 0,40 beträgt.

5. Inkontinenzartikel nach einem oder mehreren der vorstehenden Ansprüche, **dadurch gekennzeichnet, dass** der Anteil P"/N des jeweiligen durch den zweiten Fügebereich (314a, 314b, 316a, 316b) überfangenen Teilbereiches (328a, 328b, 330a, 330b) des Bauchabschnitts (4) und/oder des Rückenabschnitts (6) mit einer Breite (P") bezogen den jeweiligen Seitenbereich (360a, 360b, 362a, 362b) des Bauchabschnitts (4) und/oder des Rückenabschnitts (6) mit einer Breite (N) mindestens 0,01, insbesondere mindestens 0,015, weiter insbesondere mindestens 0,020, aber vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10 beträgt.

6. Inkontinenzartikel nach einem oder mehreren der vorstehenden Ansprüche, **dadurch gekennzeichnet, dass** der Anteil P'/N des durch einen zweiten Fügebereich (314a, 314b, 316a, 316b) überfangenen Teilbereiches (324a, 324b, 326a, 326b) des jeweiligen Überhangs (66a, 66b) mit einer Breite (P') bezogen den jeweiligen Seitenbereich (360a, 360b, 362a, 362b) des Bauchabschnitts (4) und/oder des Rückenabschnitts (6) mit einer Breite (N) mindestens 0,01, insbesondere mindestens 0,015, weiter insbesondere mindestens 0,020, aber vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10 beträgt.

7. Inkontinenzartikel nach einem oder mehreren der vorstehenden Ansprüche, **dadurch gekennzeichnet, dass** ein jeweiliger zweiter Fügebereich (314a, 314b, 316a, 316b) mit einer Gesamtbreite P derart entlang des Längsrandes (48) des Schrittabschnitts (8) angeordnet, so dass das Verhältnis der Breite P' zur Breite P" vorzugsweise zwischen 1: 4 und 4: 1, weiter vorzugsweise zwischen 1:3 und 3:1, weiter vorzugsweise zwischen 1:2 und 2:1, besonders bevorzugt 1: 1 beträgt.

8. Inkontinenzartikel nach einem oder mehreren der vorstehenden Ansprüche, **dadurch gekennzeichnet, dass** ein zweiter Fügebereich (314a, 314b, 316a, 316b) und damit ein Verstärkungsbereich {334a, 334b, 336a, 336b) eine Gesamtbreite P von 5 - 60 mm, insbesondere von 10 - 50 mm, weiter insbesondere von 10-40 mm, weiter insbesondere von 10 - 30 mm aufweist.

9. Inkontinenzartikel nach einem oder mehreren der vorstehenden Ansprüche, **dadurch gekennzeichnet, dass** ein zweiter Fügebereich (314a, 314b, 316a, 316b) parallel zur Längsrichtung (9) mit einer gleich bleibenden Gesamtbreite (P) angeordnet ist.

10. Inkontinenzartikel nach einem oder mehreren der vorstehenden Ansprüche, **dadurch gekennzeichnet, dass** die Fügemittel (340) in einem zweiten Fügebereich (314a, 314b, 316a, 316b) nicht vollflächig vorgesehen sind.

11. Inkontinenzartikel nach einem oder mehreren der vorstehenden Ansprüche, **dadurch gekennzeichnet, dass** die Fügemittel (340) in einem zweiten Fügebereich (314a, 314b, 316a, 316b) in einem Punktmuster angeordnet sind und wobei die Summe der durch die Fügemittel (340) gebundene Fläche einen Anteil von mindestens 1,5 %, insbesondere von mindestens 2,0 %, weiter insbesondere mindestens 2,3 %, weiter insbesondere mindestens 2,5 %, und vorzugsweise höchstens 20,0%, insbesondere höchstens 15,0%, weiter insbesondere höchstens 10,0%, weiter insbesondere höchstens 8,0%, weiter insbesondere höchstens 7,0 %, weiter insbesondere höchstens 6,0% bezogen auf die von einem zweiten Fügebereich überfangene Gesamtfläche einnimmt.

12. Inkontinenzartikel nach einem oder mehreren der vorstehenden Ansprüche, **dadurch gekennzeichnet, dass** der Überhang (66a, 66b) des Backsheet-Materials (62) und/oder des Dockblatt-Materials (84) in Querrichtung (16) in der Summe, also beidseits der Längsränder (46) des Absorptionskörpers (7) wenigstens 25 %, insbesondere 25 - 50 %, weiter insbesondere 30 - 45 % und weiter insbesondere 35 - 45 % bezogen auf eine größte Breite (E) des Schrittabschnitts (8) beträgt.

13. Inkontinenzartikel nach einem oder mehreren der vorstehenden Ansprüche, **dadurch gekennzeichnet, dass** sich die zweiten Elastifizierungsmittel (40, 42) ausgehend von den beiden Seitennahtbereichen (14) in Richtung (56) auf eine Längsmittelachse (44) des Inkontinenzartikels (2) erstrecken und dabei bogenförmig sich auffächernd mit zunehmendem Abstand voneinander verlaufen.

14. Inkontinenzartikel nach einem oder mehreren der

vorstehenden Ansprüche, **dadurch gekennzeichnet, dass** ein erster Fügebereich (310, 312) durch Klebemittel, insbesondere Hotmeltklebemittel gebildet ist.

15. Inkontinenzartikel nach einem oder mehreren der vorstehenden Ansprüche, **dadurch gekennzeichnet, dass** ein jeweiliger zweiter Fügebereich (314a, 314b, 316a, 316b) außerhalb der Kontur des Absorptionskörpers angeordnet ist.

## Claims

1. An incontinence article (2) in pants form for receiving body excretions, with a front stomach portion (4) and a rear back portion (6), which to form a stomach and back band which is continuous in the transverse or waist-encircling direction (16) and has a waist opening (18) that is closed in the waist-encircling direction are connected to one another at the manufacturer's at side seam regions (14) on both sides, and with a crotch portion (8), which has an absorbent body (7), and has an inner side (85), an outer side (86), with longitudinal peripheries (48) and longitudinal ends (98, 100), extends in a longitudinal direction (9) between the stomach portion (4) with an inner side (41) and with a crotch-facing transverse periphery (58) and the back portion (6) with an inner side (41) and with a crotch-facing transverse periphery (60), wherein the crotch portion (8) overlaps with the stomach portion (4) in a front overlapping region (36) and the crotch portion (8) overlaps with the back portion (6) in a rear overlapping region (38), wherein the crotch portion (8) is inseparably joined with its outer side (86) to the inner side (41) of the stomach portion (4) in a front connecting region (306) and the crotch portion (8) is inseparably joined with its outer side (86) to the inner side (41) of the back portion (6) in a rear connecting region (308), wherein not only the crotch portion (8) but also the stomach portion (4) and the back portion (6) bound the leg openings (19) of the incontinence article, wherein first elasticating means (28) are provided in the stomach portion (4) and the back portion (6), are spaced apart from one another and extend parallel to one another in the transverse or waist-encircling direction (16) and thus elasticate the stomach portion (4) and the back portion (6) over their surface areas, wherein second elasticating means (40, 42) are provided in a crotch-side region (22, 26) of the stomach portion (4) and of the back portion (6) that is facing the leg openings (19), wherein the crotch portion (8) comprises a liquid-impermeable backsheet material (62), and wherein the absorbent body (7) is arranged between the backsheet material (62) and a topsheet material (84) and the absorbent body (7) has longitudinal peripheries (46), wherein the topsheet material (84) or the topsheet material (84) and the backsheet material (62) form(s) an overhang (66a, 66b) respectively extending outside the longitudinal peripheries (46) of the absorbent body (7), wherein the connecting regions (306, 308) of the stomach portion (4) and of the back portion (6) respectively comprise a first joining region (310, 312) and second joining regions (314a, 314b, 316a, 316b), wherein the first joining region (310, 312) extends at least in certain portions beneath the absorbent body (7), and wherein the second joining regions (314a, 314b, 316a, 316b) are provided in a region (320a, 320b, 322a, 322b) bridging the respective longitudinal periphery (48) of the crotch portion (8), that is to say both a subregion (324a, 324b, 326a, 326b) of the overhang (66a, 66b) of the crotch portion (8) and a subregion (328a, 328b, 330a, 330b) adjacent thereto of the stomach portion (4) and/or of the back portion (6) are extended over, and wherein a respective second joining region (314a, 314b, 316a, 316b) extends in the longitudinal direction (9) respectively from a crotch-facing transverse periphery (58, 60) of the stomach portion (4) and/or of the back portion (6) in the direction of the longitudinal ends (98, 100) of the crotch portion (8), and wherein a respective second joining region (314a, 314b, 316a, 316b) is formed by joining means (340) in the form of welding locations, in particular ultrasonic welding locations, thermal welding locations and/or calender welding locations, so that the second joining regions (314a, 314b, 316a, 316b) form reinforcing regions (334a, 334b, 336a, 336b).

2. The incontinence article as claimed in claim 1, **characterized in that** the second joining regions (314a, 314b, 316a, 316b), and consequently the reinforcing regions (334a, 334b, 336a, 336b), extend, when considered in the longitudinal direction (9), from the crotch-facing transverse periphery (58, 60) of the stomach portion (4) and of the back portion (6) to at least the respective longitudinal end (98, 100) of the crotch portion (8).

3. The incontinence article as claimed in one or more of the preceding claims, **characterized in that** the proportion P'/H of the subregion (324a, 324b, 326a, 326b) of the respective overhang (66a, 66b) that is extended over by a second joining region (314a, 314b, 316a, 316b), with a width (P') with respect to the respective overhang (66a, 66b) with a width (H) in the front and/or rear overlapping region (36, 38), is at least 0.01, particularly at least 0.04, more particularly at least 0.07, more particularly at least 0.10, but preferably at most 0.90, particularly at most 0.80, more particularly at most 0.70, more particularly at most 0.60, more particularly at most 0.50, more particularly at most 0.40.

4. The incontinence article as claimed in one or more of the preceding claims, **characterized in that** the proportion P"/H of the respective subregion (328a, 328b, 330a, 330b) of the stomach portion (4) and/or of the back portion (6) that is extended over by a second joining region (314a, 314b, 316a, 316b), with a width (P") with respect to the respective overhang (66a, 66b) with a width (H), is at least 0.01, particularly at least 0.04, more particularly at least 0.07, more particularly at least 0.10, but preferably at most 0.90, more particularly at most 0.80, more particularly at most 0.70, more particularly at most 0.60, more particularly at most 0.50, more particularly at most 0.40.

5. The incontinence article as claimed in one or more of the preceding claims, **characterized in that** the proportion P"/N of the respective subregion (328a, 328b, 330a, 330b) of the stomach portion (4) and/or of the back portion (6) that is extended over by the second joining region (314a, 314b, 316a, 316b), with a width (P") with respect to the respective side region (360a, 360b, 362a, 362b) of the stomach portion (4) and/or of the back portion (6) with a width (N), is at least 0.01, particularly at least 0.015, more particularly at least 0.020, but preferably at most 0.35, particularly at most 0.30, more particularly at most 0.25, more particularly at most 0.20, more particularly at most 0.15, more particularly at most 0.10.

6. The incontinence article as claimed in one or more of the preceding claims, **characterized in that** the proportion P'/N of the subregion (324a, 324b, 326a, 326b) of the respective overhang (66a, 66b) that is extended over by a second joining region (314a, 314b, 316a, 316b), with a width (P') with respect to the respective side region (360a, 360b, 362a, 362b) of the stomach portion (4) and/or of the back portion (6) with a width (N), is at least 0.01, particularly at least 0.015, more particularly at least 0.020, but preferably at most 0.35, particularly at most 0.30, more particularly at most 0.25, more particularly at most 0.20, more particularly at most 0.15, more particularly at most 0.10.

7. The incontinence article as claimed in one or more of the preceding claims, **characterized in that** a respective second joining region (314a, 314b, 316a, 316b) with an overall width P is arranged along the longitudinal periphery (48) of the crotch portion (8) in such a way that the ratio of the width P' to the width P" is preferably between 1:4 and 4:1, more preferably between 1:3 and 3:1, more preferably between 1:2 and 2:1, particularly preferably 1:1.

8. The incontinence article as claimed in one or more of the preceding claims, **characterized in that** a second joining region (314a, 314b, 316a, 316b), and consequently a reinforcing region (334a, 334b, 336a, 336b), has an overall width P of 5 - 60 mm, particularly of 10 - 50 mm, more particularly of 10 - 40 mm, more particularly of 10 - 30 mm.

9. The incontinence article as claimed in one or more of the preceding claims, **characterized in that** a second joining region (314a, 314b, 316a, 316b) is arranged parallel to the longitudinal direction (9) with a constant overall width (P).

10. The incontinence article as claimed in one or more of the preceding claims, **characterized in that** the joining means (340) in a second joining region (314a, 314b, 316a, 316b) are not provided over the full surface area.

11. The incontinence article as claimed in one or more of the preceding claims, **characterized in that** the joining means (340) in a second joining region (314a, 314b, 316a, 316b) are arranged in a point pattern and the sum of the surface area attached by the joining means (340) assumes a proportion of at least 1.5%, particularly of at least 2.0%, more particularly at least 2.3%, more particularly at least 2.5%, and preferably at most 20.0%, particularly at most 15.0%, more particularly at most 10.0%, more particularly at most 8.0%, more particularly at most 7.0%, more particularly at most 6.0%, with respect to the overall surface area extended over by a second joining region.

12. The incontinence article as claimed in one or more of the preceding claims, **characterized in that** the overhang (66a, 66b) of the backsheet material (62) and/or of the topsheet material (84) in the transverse direction (16) is in total, that is to say on both sides of the longitudinal peripheries (46) of the absorbent body (7), at least 25%, particularly 25 - 50%, more particularly 30 - 45% and more particularly 35 - 45%, with respect to a greatest width (E) of the crotch portion (8).

13. The incontinence article as claimed in one or more of the preceding claims, **characterized in that** the second elasticating means (40, 42) extend from the two side seam regions (14) in the direction (56) of a longitudinal center axis (44) of the incontinence article (2) and thereby run in an arcuately fanning-out manner with increasing distance from one another.

14. The incontinence article as claimed in one or more of the preceding claims, **characterized in that** a first joining region (310, 312) is formed by adhesive, in particular hotmelt adhesive.

15. The incontinence article as claimed in one or more of the preceding claims, **characterized in that** a respective second joining region (314a, 314b, 316a, 316b) is arranged outside the contour of the absorbent body.

## Revendications

1. Article d'incontinence (2) en forme de slip, destiné à reprendre des déjections corporelles et présentant une partie ventrale avant (4) et une partie dorsale arrière (6) qui, pour former une bande ventrale et dorsale continue dans la direction transversale ou de tour de hanche (16) sont reliées l'une à l'autre lors de la fabrication, avec une ouverture de hanche (18), fermée dans la direction du tour de hanche, sur des parties (14) de couture latérale situées des deux côtés,
une partie d'entrejambe (8) présentant un corps d'absorption (7) et dotée d'un côté intérieur (85), d'un côté extérieur (86), de bords longitudinaux (48) et d'extrémités longitudinales (98, 100) et qui s'étend dans une direction longitudinale (9) entre la partie ventrale (4) et la partie de dos (6) par un côté intérieur (41) et un bord transversal (58) tourné vers l'entrejambe et par un côté intérieur (41) et un bord transversal (60) tourné vers l'entrejambe,
la partie d'entrejambe (8) se superposant à la partie ventrale (4) dans une partie (36) de superposition avant et la partie d'entrejambe (8) se superposant à la partie dorsale (6) dans une zone de superposition arrière (38),
le côté extérieur (86) de la partie d'entrejambe (8) étant joint de manière non libérable au côté intérieur (41) de la partie ventrale (4) dans une zone (306) de liaison avant et le côté extérieur (86) de la partie d'entrejambe (8) étant joint de manière non libérable au côté intérieur (41) de la partie de dos (6) dans une zone (308) de liaison arrière,
la partie d'entrejambe (8), la partie ventrale (4) et la partie dorsale (6) délimitant les ouvertures de jambes (19) de l'article d'incontinence,
la partie ventrale (4) et la partie dorsale (6) présentant des premiers moyens d'élastification (28) qui s'étendent à distance mutuelle et parallèlement l'un à l'autre dans la direction transversale ou de tour de hanche (16) et élastifient ainsi la partie ventrale (4) et la partie dorsale (6) sur toute leur surface,
des deuxièmes moyens d'élastification (40, 42) étant prévus dans une zone (22, 26), située au niveau de l'entrejambe et tournée vers les ouvertures de jambes (19), de la partie ventrale (4) et de la partie dorsale (6),
la partie d'entrejambe (8) comportant un matériau (62) de feuille de dos perméable aux liquides,
le corps d'absorption (7) étant disposé entre le matériau (62) de feuille de dos et un matériau (84) de feuille de couverture et le corps d'absorption (7) présentant des bords longitudinaux (46),
le matériau (84) de feuille de recouvrement ou le matériau (84) de feuille de recouvrement et le matériau (62) de feuille de dos formant dans la direction transversale (16) des porte-à-faux (66a, 66b) qui s'étendent tous deux à l'extérieur des bords longitudinaux (46) du corps d'absorption (7),
les zones de liaison (306, 308) de la partie ventrale (4) et de la partie dorsale (6) comportant chacune une première zone de jonction (310, 312) et des deuxièmes zones de jonction (314a, 314b, 316a, 316b),
au moins certaines parties de la première zone de jonction (310, 312) s'étendant en dessous du corps d'absorption (7),
les deuxièmes zones de jonction (314a, 314b, 316a, 316b) étant prévues dans une zone (320a, 320b, 322a, 322b) qui recouvre le bord longitudinal (48) concerné de la partie d'entrejambe (8) et reprenant ainsi à la fois une partie (324a, 324b, 326a, 326b) du porte-à-faux (66a, 66b) de la partie d'entrejambe (8) et une partie (328a, 328b, 330a, 330b), qui lui est adjacente, de la partie ventrale (4) et/ou de la partie dorsale (6),
une deuxième zone de jonction respective (314a, 314b, 316a, 316b) s'étendant dans le sens longitudinal (9) en commençant par un bord transversal (58, 60), tourné vers l'entrejambe, de la partie ventrale (4) et/ou de la partie dorsale (6) en direction des extrémités longitudinales (98, 100) de la partie d'entrejambe (8),
une deuxième zone de jonction respective (314a, 314b, 316a, 316b) étant formée par des moyens de jonction (340) qui présentent la forme d'emplacements soudés, en particulier d'emplacements soudés par ultrasons, d'emplacements soudés thermiquement et/ou d'emplacements soudés à la calandre, de sorte que les deuxièmes zones de jonction (314a, 314b, 316a, 316b) forment des zones de renfort (334a, 334b, 336a, 336b).

2. Article d'incontinence selon la revendication 1, **caractérisé en ce que** les deuxièmes zones de jonction (314a, 314b, 316a, 316b) et donc les zones de renfort (334a, 334b, 336a, 336b) s'étendent dans le sens de la longueur (9) en partant du bord transversal (58, 60), tourné vers l'entrejambe, de la partie ventrale (4) et de la partie dorsale (6) au moins jusqu'à l'extrémité longitudinale respective (98, 100) de la partie d'entrejambe (8).

3. Article d'incontinence selon l'une ou plusieurs des revendications précédentes, **caractérisé en ce que** dans la zone de superposition avant et/ou la zone de superposition arrière (36, 38), le rapport P'/H entre la partie (324a, 324b, 326a, 326b) de largeur (P') reprise par une deuxième zone de jonction (314a, 314b, 316a, 316b) de chaque porte-à-faux (66a, 66b) et le porte-à-faux (66a, 66b) respectif de largeur (H) est d'au moins 0,01, en particulier d'au moins 0,04, plus particulièrement d'au moins 0,07, plus particulièrement d'au moins 0,10, et plus particulièrement d'au plus 0,90, en particulier d'au plus 0,80, plus particulièrement d'au plus 0,70, plus particulièrement d'au plus 0,60, plus particulièrement d'au plus 0,50 et plus particulièrement d'au plus 0,40.

4. Article d'incontinence selon l'une ou plusieurs des revendications précédentes, **caractérisé en ce que** le rapport P''/H entre chaque partie (328a, 328b, 330a, 330b) de largeur (P'') reprise par une deuxième zone de jonction (314a, 314b, 316a, 316b), de la partie ventrale (4) et/ou de la partie dorsale (6) et le porte-à-faux respectif (66a, 66b) de largeur (H) est d'au moins 0,01, en particulier d'au moins 0,04, plus particulièrement d'au moins 0,07, plus particulièrement d'au moins 0,10, et plus particulièrement d'au plus 0,90, en particulier d'au plus 0,80, plus particulièrement d'au plus 0,70, plus particulièrement d'au plus 0,60, plus particulièrement d'au plus 0,50 et plus particulièrement d'au plus 0,40.

5. Article d'incontinence selon l'une ou plusieurs des revendications précédentes, **caractérisé en ce que** le rapport P''/N entre la partie (328a, 328b, 330a, 330b) de largeur (P'') reprise par la deuxième zone de jonction (314a, 314b, 316a, 316b), de la partie ventrale (4) et/ou de la partie dorsale (6) et la partie latérale respective (360a, 360b, 362a, 362b) de largeur (N) de la partie ventrale (4) et/ou de la partie dorsale (6) est d'au moins 0,01, en particulier d'au moins 0,015, de manière plus particulière d'au moins 0,020 et de préférence d'au plus 0,35, en particulier d'au plus 0,30, de manière plus particulière d'au plus 0,25, de manière plus particulière d'au plus 0,20, de manière plus particulière d'au plus 0,15 et de manière plus particulière d'au plus 0,10.

6. Article d'incontinence selon l'une ou plusieurs des revendications précédentes, **caractérisé en ce que** le rapport P'/N entre la partie (324a, 324b, 326a, 326b) de largeur (P') reprise par une deuxième zone de jonction (314a, 314b, 316a, 316b) du porte-à-faux respectif (66a, 66b) et la partie latérale respective (360a, 360b, 362a, 362b) de largeur (N) de la partie ventrale (4) et/ou de la partie dorsale (6) est d'au moins 0,01, en particulier d'au moins 0,015, de manière plus particulière d'au moins 0,020 et de préférence d'au plus 0,35, en particulier d'au plus 0,30, de manière plus particulière d'au plus 0,25, de manière plus particulière d'au plus 0,20, de manière plus particulière d'au plus 0,15 et de manière plus particulière d'au plus 0,10.

7. Article d'incontinence selon l'une ou plusieurs des revendications précédentes, **caractérisé en ce qu'**une deuxième zone de jonction respective (314a, 314b, 316a, 316b) de largeur totale P est disposée le long du bord longitudinal (48) de la partie d'entrejambe (8) de telle sorte que le rapport entre la largeur P' et la largeur P'' soit de préférence compris entre 1:4 et 4:1, de manière plus préférable entre 1:3 et 3:1, de manière plus préférable entre 1:2 et 2:1 et soit de préférence de 1:1.

8. Article d'incontinence selon l'une ou plusieurs des revendications précédentes, **caractérisé en ce qu'**une deuxième zone de jonction (314a, 314b, 316a, 316b) et donc une zone de renfort (334a, 334b, 336a, 336b) présente une largeur totale P de 5 à 60 mm, en particulier de 10 à 50 mm, de manière plus particulière de 10 à 40 mm et de manière encore plus particulière de 10 à 30 mm.

9. Article d'incontinence selon l'une ou plusieurs des revendications précédentes, **caractérisé en ce qu'**une deuxième zone de jonction (314a, 314b, 316a, 316b) est disposée parallèlement au sens de la longueur (9) avec une largeur totale (P) constante.

10. Article d'incontinence selon l'une ou plusieurs des revendications précédentes, **caractérisé en ce que** les moyens de jonction (340) ne sont pas prévus sur toute la surface d'une deuxième zone de jonction (314a, 314b, 316a, 316b).

11. Article d'incontinence selon l'une ou plusieurs des revendications précédentes, **caractérisé en ce que** les moyens de jonction (340) sont disposés suivant un motif de points dans une deuxième zone de jonction (314a, 314b, 316a, 316b), la somme des surfaces liées par les moyens de jonction (340) représentant une fraction d'au moins 1,5 %, en particulier d'au moins 2,0 %, de manière plus particulière d'au moins 2,3 %, de manière plus particulière d'au moins 2,5 %, et de préférence d'au plus 20,0 %, en particulier d'au plus 15,0 %, de manière plus particulière d'au plus 10,0 %, de manière plus particulière d'au plus 8,0 %, de manière plus particulière d'au plus 7,0 %, de manière plus particulière d'au plus 6,0 %, par rapport à la surface totale reprise par une deuxième zone de jonction.

12. Article d'incontinence selon l'une ou plusieurs des revendications précédentes, **caractérisé en ce que** dans la direction transversale (16), et donc des deux côtés des bords longitudinaux (46) du corps d'absorption (7), le porte-à-faux (66a, 66b) du matériau (62) de feuille de dos et/ou de matériau (84) de feuille de recouvrement vaut au total au moins 25 %, en particulier de 25 à 50 %, de manière plus particulière de 30 à 45 % et de manière plus particulière de 35 à 45 % par rapport à la plus grande largeur (E) de la partie d'entrejambe (8).

13. Article d'incontinence selon l'une ou plusieurs des revendications précédentes, **caractérisé en ce que** les deuxièmes moyens d'élastification (40, 42) partent des deux zones (14) de cordon latéral pour s'étendre dans la direction (56) sur un axe longitudinal central (44) de l'article d'incontinence (2) et s'étendent ainsi à une distance croissante l'un de l'autre en s'évasant en arc de cercle.

**14.** Article d'incontinence selon l'une ou plusieurs des revendications précédentes, **caractérisé en ce qu'**une première zone de jonction (310, 312) est formée par des moyens adhésifs et en particulier des moyens adhésifs fusibles à chaud.

**15.** Article d'incontinence selon l'une ou plusieurs des revendications précédentes, **caractérisé en ce que** chaque deuxième zone de jonction (314a, 314b, 316a, 316b) est disposée à l'extérieur du contour du corps d'absorption.

Fig. 1

Fig. 2

314b

4

350

340, 342

IV

344

8

Fig. 3

66b

62 84

340, 342

344

48

340, 342

4

Fig. 4

Fig. 5

Fig. 6

Fig. 7

Fig. 8

Fig. 9

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004052260 A1
- WO 03039423 A1
- WO 2005067842 A1
- WO 2005016200 A1
- EP 1392212 B1